# EUROPEAN PATENT SPECIFICATION

(11) **EP 4 171 360 B1**
(45) Date of publication and mention of the grant of the patent: **31.07.2024**
(21) Application number: 21748711.5
(22) Date of filing: 29.06.2021
(51) Int. Cl.: A61B 5/00, A61B 5/087, G16H 20/70, G16H 50/30, G16H 40/67

(54) **SYSTEMS AND METHODS FOR MULTI-COMPONENT HEALTH SCORING**
SYSTEME UND VERFAHREN ZUR GESUNDHEITSBEWERTUNG AUS MEHREREN KOMPONENTEN
SYSTÈMES ET PROCÉDÉS DE NOTATION DE LA SANTÉ À PLUSIEURS COMPOSANTES

(30) Priority: 30.06.2020 US 202063046259 P; 30.06.2020 US 202063046281 P; 30.06.2020 US 202063046299 P
(43) Date of publication of application: 03.05.2023
(73) Proprietor: ResMed Sensor Technologies Limited, Sandyford D18 T6T7 Dublin (IE)
(72) Inventor: MOLONY, Katherine, Bella Vista, New South Wales 2153 (AU); BEADLE, Dylan, Hermes da Fonseca, San Diego, California 92123 (US); CERINA, Luca, Dublin, D18 T6T7 (IE); VITHANAGE FERNANDO, Varuni Lakshana, Bella Vista, New South Wales 2153 (AU); SHOULDICE, Redmond, Dublin, D18 T6T7 (IE); XU, Jessica, Dublin, D18 T6T7 (IE); GUPTA, Priyanshu, Bella Vista, New South Wales 2153 (AU)
(74) Representative: Mathys & Squire
(86) International application number: PCT/US2021/039721
(87) International publication number: WO 2022/006183

(56) References cited:
- WO-A1-2019/122412
- US-A1- 2015 112 722
- US-A1- 2019 355 271

## Description

### CROSS-REFERENCE TO RELATED APPLICATIONS

This application claims the benefit of, and priority to, U.S. Provisional Patent Application No. 63/046,259, filed June 30, 2020, U.S. Provisional Patent Application No. 63/046,281, filed June 30, 2020, and U.S. Provisional Patent Application No. 63/046,299, filed June 30, 2020.

### TECHNICAL FIELD

The present disclosure relates generally to systems and methods for encouraging a behavioral response by a user, and more particularly, to systems and methods for generating a multi-component health score to aid in encouraging a behavioral response.

### BACKGROUND

Many individuals suffer from sleep-related and/or respiratory-related disorders such as, for example, Periodic Limb Movement Disorder (PLMD), Restless Leg Syndrome (RLS), Sleep-Disordered Breathing (SDB) such as Obstructive Sleep Apnea (OSA), Central Sleep Apnea (CSA), other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders. These disorders are often treated using a respiratory therapy system. However, some users find such systems to be uncomfortable, difficult to use, expensive, aesthetically unappealing and/or fail to perceive the benefits associated with using the system.

As a result, some users will elect not to begin using the respiratory therapy system or discontinue use of the respiratory therapy system absent a demonstration of the severity of their symptoms when respiratory therapy treatment is not used. The present disclosure is directed to solving these and other problems.

Document WO 2019/122412 A1 discloses a system for use by a patient benefiting at nigh from a continuous positive airway pressure CPAP therapy device. The system is configured to use data acquired by CPAP sensors during the night as well as daytime parameters such as step counts and/or exercise intensity readings to formulate a wellness score related to a previous night sleep score. The system may also be configured to ask subjective questions to the patient during the day to elucidate the patient's relative feeling of alertness of sleepiness.

### SUMMARY

The invention is defined by the appended claims.

According to some implementations of the present disclosure, a method includes receiving physiological data associated with a sleep session, the physiological data including first physiological data collected during the sleep session and second physiological data collected during a duration adjacent the sleep session. The method also includes receiving subjective feedback data associated with the sleep session. The method also includes generating a set of component scores based on the physiological data and the subjective feedback data. The method also includes calculating a total health score associated with the sleep session using the set of component scores. The method also includes presenting the total health score.

According to some implementations of the present disclosure, a method includes receiving a set of existing component scores associated with a sleep session or a previous sleep session. The method also includes receiving data associated with the sleep session. The receiving data associated with the sleep session includes receiving physiological data associated with the sleep session. The physiological data includes first physiological data collected during the sleep session and second physiological data collected during a duration adjacent the sleep session. Alternatively or additionally, the receiving data associated with the sleep session includes receiving subjective feedback data associated with the sleep session. The method also includes generating a set of updated component scores based on the set of existing component scores and the received data. The method also includes calculating a total health score associated with the sleep session using the set of updated component scores. The method also includes presenting the total health score.

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system, according to some implementations of the present disclosure;
FIG. 2 is a perspective view of at least a portion of the system of FIG. 1, a user, and a bed partner, according to some implementations of the present disclosure;
FIG. 3 illustrates an exemplary timeline for a sleep session, according to some implementations of the present disclosure;
FIG. 4 illustrates an exemplary hypnogram associated with the sleep session of FIG. 3, according to some implementations of the present disclosure;
FIG. 5A illustrates a first portion of an exemplary dashboard view displayed on a display device, according to some implementations of the present disclosure;
FIG. 5B illustrates a second portion of the exemplary dashboard view of FIG. 5A, according to some implementations of the present disclosure;
FIG. 5C illustrates a calendar portion of the dashboard view of FIG. 5A, according to some implementations of the present disclosure;
FIG. 5D illustrates a total health score portion of the dashboard view of FIG. 5A, according to some implementations of the present disclosure;
FIG. 5E illustrates a blood pressure measurement portion of the dashboard view of FIG. 5A, according to some implementations of the present disclosure;
FIG. 6 illustrates a total health score calculation view displayed on a display device, according to some implementations of the present disclosure;
FIG. 7A illustrates a first blood pressure measurement view displayed on a display device, according to some implementations of the present disclosure;
FIG. 7B illustrates a second blood pressure measurement view displayed on a display device, according to some implementations of the present disclosure;
FIG. 7C illustrates a blood pressure health threshold view displayed on a display device, according to some implementations of the present disclosure;
FIG. 8A illustrates a trend view displayed on a display device, according to some implementations of the present disclosure;
FIG. 8B illustrates the trend view of FIG. 8A and a day indicator displayed on the display device, according to some implementations of the present disclosure;
FIG. 9 illustrates exemplary fitted trend lines for a first parameter, according to some implementations of the present disclosure;
FIG. 10A illustrates a sleep score portion of the trend view of FIG. 8A, according to some implementations of the present disclosure;
FIG. 10B illustrates a blood pressure measurement portion of the trend view of FIG. 8A, according to some implementations of the present disclosure;
FIG. 11 is a flowchart depicting a process for determining a health score, according to certain aspects of the present disclosure;
FIG. 12 is a flowchart depicting a process for calculating a health score from component scores, according to certain aspects of the present disclosure;
FIG. 13 is a process flow diagram illustrating a method for generating a custom message, according to some implementations of the present disclosure;
FIG. 14 is a process flow diagram illustrating a method for prioritizing a custom message to be communicated to a user, according to some implementations of the present disclosure; and
FIG. 15 illustrates exemplary data points for a plurality of parameters during a time period, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, and alternatives falling within the scope of the present disclosure as defined by the appended claims

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB) such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hyper tension, diabetes, stroke, insomnia, and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as Central Sleep Apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathin. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

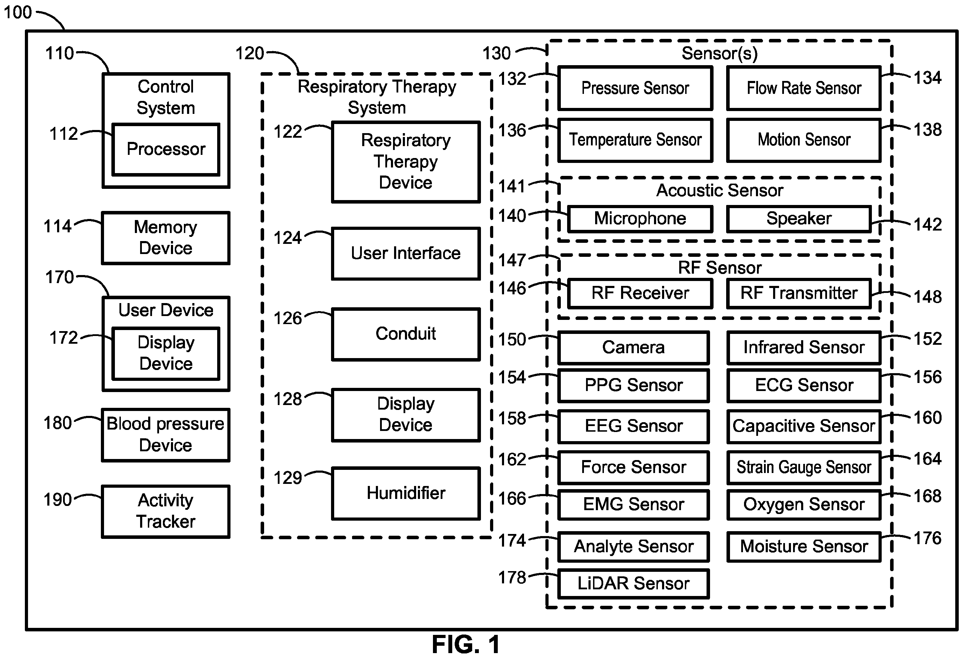

Referring to FIG. 1, a system 100, according to some implementations of the present disclosure, is illustrated. The system 100 includes a control system 110, a memory device 114, one or more sensors 130, and one or more user devices 170. In some implementations, the system 100 further optionally includes a respiratory therapy system 120, a blood pressure device 180, an activity tracker 190, or any combination thereof.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is illustrated in FIG. 1, the control system 110 can include any number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the user device 170, a portion (e.g., the respiratory therapy device 122) of the respiratory therapy system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

As described herein, the processor 112 of the control system 110 and/or memory device 114 can receive data (e.g., physiological data and/or audio data) from the one or more sensors 130 such that the data for storage in the memory device 114 and/or for analysis by the processor 112. The processor 112 and/or memory device 114 can communicate with the one or more sensors 130 using a wired connection or a wireless connection (e.g., using an RF communication protocol, a Wi-Fi communication protocol, a Bluetooth communication protocol, over a cellular network, etc.). In some implementations, the system 100 can include an antenna, a receiver (e.g., an RF receiver), a transmitter (e.g., an RF transmitter), a transceiver, or any combination thereof. Such components can be coupled to or integrated a housing of the control system 110 (e.g., in the same housing as the processor 112 and/or memory device 114), or the user device 170.

As noted above, in some implementations, the system 100 optionally includes a respiratory therapy system 120 (also referred to as a respiratory therapy system). The respiratory therapy system 120 can include a respiratory pressure therapy device 122 (referred to herein as respiratory therapy device 122), a user interface 124 (also referred to as a mask or patient interface), a conduit 126 (also referred to as a tube or an air circuit), a display device 128, a humidifier 129, or any combination thereof. In some implementations, the control system 110, the memory device 114, the display device 128, one or more of the sensors 130, and the humidifier 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory therapy device 122 is generally used to generate pressurized air that is delivered to a user (e.g., using one or more motors that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Generally, the user interface 124 engages the user's face such that the pressurized air is delivered to the user's airway via the user's mouth, the user's nose, or both the user's mouth and nose. Together, the respiratory therapy device 122, the user interface 124, and the conduit 126 form an air pathway fluidly coupled with an airway of the user. The pressurized air also increases the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cm H₂O.

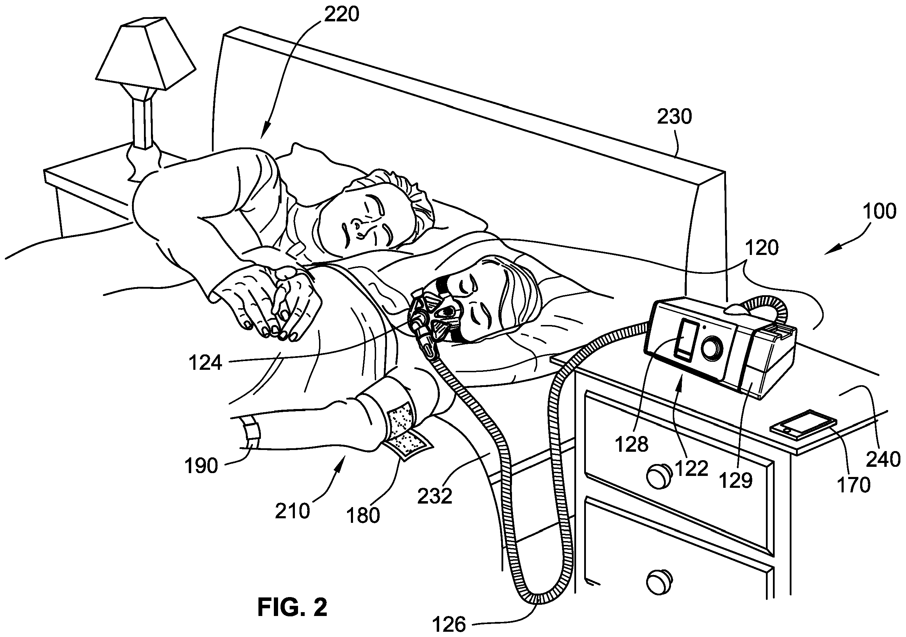

As shown in FIG. 2, in some implementations, the user interface 124 is a facial mask that covers the nose and mouth of the user. Alternatively, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 is a mouthpiece (e.g., a night guard mouthpiece molded to conform to the user's teeth, a mandibular repositioning device, etc.) for directing pressurized air into the mouth of the user.

The conduit 126 (also referred to as an air circuit or tube) allows the flow of air between two components of a respiratory therapy system 120, such as the respiratory therapy device 122 and the user interface 124. In some implementations, there can be separate limbs of the conduit for inhalation and exhalation. In other implementations, a single limb conduit is used for both inhalation and exhalation. The conduit 126 includes a first end coupled to an outlet of the respiratory therapy device 122 and a second opposing end coupled to the user interface 124. The conduit 126 can be coupled to the respiratory therapy device 122 and/or the user interface 124 using a variety of techniques (e.g., a press fit connection, a snap fit connection, a threaded connection, etc.). In some implementations, the conduit 126 includes one or more heating elements that heat the pressurized air flowing through the conduit 126 (e.g., heat the air to a predetermined temperature or within a range of predetermined temperatures). Such heating elements can be coupled to and/or imbedded in the conduit 126. In such implementations, the end of the conduit 126 coupled to the respiratory therapy device 122 can include an electrical contact that is electrically coupled to the respiratory therapy device 122 to power the one or more heating elements of the conduit 126.

One or more of the respiratory therapy device 122, the user interface 124, the conduit 126, the display device 128, and the humidifier 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score (also referred to as a myAir score, such as described in WO 2016/061629 and U.S. Patent Pub. No. 2017/0311879), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidifier 129 is coupled to or integrated in the respiratory therapy device 122 and includes a reservoir of water that can be used to humidify the pressurized air delivered from the respiratory therapy device 122. The respiratory therapy device 122 can include a heater to heat the water in the humidifier 129 in order to humidify the pressurized air provided to the user. Additionally, in some implementations, the conduit 126 can also include a heating element (e.g., coupled to and/or imbedded in the conduit 126) that heats the pressurized air delivered to the user.

The respiratory therapy system 120 can be used, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can be positioned on a nightstand 240 that is directly adjacent to the bed 230 as shown in FIG. 2, or more generally, on any surface or structure that is generally adjacent to the bed 230 and/or the user 210.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example, physiological data, audio data, or both. Physiological data generated by one or more of the sensors 130 can be used by the control system 110 to determine a sleep-wake signal associated with a user during a sleep session and one or more sleep-related parameters. The sleep-wake signal can be indicative of one or more sleep states, including wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof. Methods for determining sleep states and/or sleep stages from physiological data generated by one or more sensors, such as the one or more sensors 130, are described in, for example, WO 2014/047310, U.S. Patent Pub. No. 2014/0088373, WO 2017/132726, WO 2019/122413, WO 2019/122414, and U.S. Patent Pub. No. 2020/0383580. The sleep-wake signal can be measured by the sensor(s) 130 during the sleep session at a predetermined sampling rate, such as, for example, one sample per second, one sample per 30 seconds, one sample per minute, etc. Examples of the one or more sleep-related parameters that can be determined for the user during the sleep session based on the sleep-wake signal include a total time in bed, a total sleep time, a sleep onset latency, a wake-after-sleep-onset parameter, a sleep efficiency, a fragmentation index, or any combination thereof.

The sleep-wake signal can also be timestamped to indicate a time that the user enters the bed, a time that the user exits the bed, a time that the user attempts to fall asleep, etc. The sleep-wake signal can be measured by the one or more sensors 130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory therapy device 122, or any combination thereof during the sleep session. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof. The one or more sleep-related parameters that can be determined for the user during the sleep session based on the sleep-wake signal include, for example, a total time in bed, a total sleep time, a sleep onset latency, a wake-after-sleep-onset parameter, a sleep efficiency, a fragmentation index, or any combination thereof. As described in further detail herein, the physiological data and/or the sleep-related parameters can be analyzed to determine one or more sleep-related scores.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, pressure settings of the respiratory therapy device 122, or any combination thereof. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. Examples of flow rate sensors (such as, for example, the flow rate sensor 134) are described in International Publication No. WO 2012/012835 and U.S. Patent No. 10,328,219.

In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature of the air flowing from the respiratory therapy device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user 210 during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. In some implementations, the motion sensor 138 alternatively or additionally generates one or more signals representing bodily movement of the user, from which may be obtained a signal representing a sleep state of the user; for example, via a respiratory movement of the user. In some implementations, the motion data from the motion sensor 138 can be used in conjunction with additional data from another one of the sensors 130 to determine the sleep state of the user.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory therapy device 122, the user interface 124, the conduit 126, or the user device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 outputs sound waves that are audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein such as, for example, a respiration signal, a respiration rate, an inspiration amplitude, an expiration amplitude, an inspiration-expiration ratio, a number of events per hour, a pattern of events, a sleep state, a sleep stage, pressure settings of the respiratory therapy device 122, or any combination thereof. In such a context, a sonar sensor may be understood to concern an active acoustic sensing, such as by generating and/or transmitting ultrasound and/or low frequency ultrasound sensing signals (e.g., in a frequency range of about 17-23 kHz, 18-22 kHz, or 17-18 kHz, for example), through the air.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication can be Wi-Fi, Bluetooth, or the like.

In some implementations, the RF sensor 147 is a part of a mesh system. One example of a mesh system is a Wi-Fi mesh system, which can include mesh nodes, mesh router(s), and mesh gateway(s), each of which can be mobile/movable or fixed. In such implementations, the Wi-Fi mesh system includes a Wi-Fi router and/or a Wi-Fi controller and one or more satellites (e.g., access points), each of which include an RF sensor that the is the same as, or similar to, the RF sensor 147. The Wi-Fi router and satellites continuously communicate with one another using Wi-Fi signals. The Wi-Fi mesh system can be used to generate motion data based on changes in the Wi-Fi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or any combination thereof. Further, the image data from the camera 150 can be used to, for example, identify a location of the user, to determine chest movement of the user 210 (FIG. 2), to determine air flow of the mouth and/or nose of the user 210, to determine a time when the user 210 enters the bed 230 (FIG. 2), and to determine a time when the user 210 exits the bed 230. In some implementations, the camera 150 includes a wide angle lens or a fish eye lens. For example, the image data from the camera 150 can be used to identify a location of the user, to determine a time when the user 210 enters the bed 230 (FIG. 2), and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory therapy device 122, etc.). Thus, in some implementations, the moisture sensor 176 can be coupled to or integrated in the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, a rain sensor, a soil moisture sensor, a water flow sensor, an alcohol sensor, or any combination thereof.

While shown separately in FIG. 1, any combination of the one or more sensors 130 can be integrated in and/or coupled to any one or more of the components of the system 100, including the respiratory therapy device 122, the user interface 124, the conduit 126, the humidifier 129, the control system 110, the user device 170, or any combination thereof. For example, the microphone 140 and speaker 142 is integrated in and/or coupled to the user device 170 and the pressure sensor 132 and/or flow rate sensor 134 are integrated in and/or coupled to the respiratory therapy device 122. In some implementations, at least one of the one or more sensors 130 is not coupled to the respiratory therapy device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

One or more of the respiratory therapy device 110, the user interface 124, the conduit 126, the display device 128, and the humidifier 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, a sleep state, an apnea-hypopnea index (AHI), or any combination thereof. The one or more events can include snoring, apneas, central apneas, obstructive apneas, mixed apneas, hypopneas, a mask leak, a cough, a restless leg, a sleeping disorder, choking, an increased heart rate, labored breathing, an asthma attack, an epileptic episode, a seizure, increased blood pressure, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 (FIG. 1) includes a display device 172. The user device 170 can be, for example, a mobile device such as a smart phone, a tablet, a gaming console, a smart watch, a laptop, or the like. Alternatively, the user device 170 can be an external sensing system, a television (e.g., a smart television) or another smart home device (e.g., a smart speaker(s) such as Google Home, Amazon Echo, Alexa etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

The blood pressure device 180 is generally used to aid in generating physiological data for determining one or more blood pressure measurements associated with a user. The blood pressure device 180 can include at least one of the one or more sensors 130 to measure, for example, a systolic blood pressure component and/or a diastolic blood pressure component.

In some implementations, the blood pressure device 180 is a sphygmomanometer including an inflatable cuff that can be worn by a user and a pressure sensor (e.g., the pressure sensor 132 described herein). For example, as shown in the example of FIG. 2, the blood pressure device 180 can be worn on an upper arm of the user 210. In such implementations where the blood pressure device 180 is a sphygmomanometer, the blood pressure device 180 also includes a pump (e.g., a manually operated bulb) for inflating the cuff. In some implementations, the blood pressure device 180 is coupled to the respiratory therapy device 122 of the respiratory therapy system 120, which in turn delivers pressurized air to inflate the cuff. More generally, the blood pressure device 180 can be communicatively coupled with, and/or physically integrated in (e.g., within a housing), the control system 110, the memory 114, the respiratory therapy system 120, the user device 170, and/or the activity tracker 190.

The activity tracker 190 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum he respiration art rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), or any combination thereof. The activity tracker 190 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 190 is a wearable device that can be worn by the user, such as a smartwatch, a wristband, a ring, or a patch. For example, referring to FIG. 2, the activity tracker 190 is worn on a wrist of the user 210. The activity tracker 190 can also be coupled to or integrated a garment or clothing that is worn by the user. Alternatively still, the activity tracker 190 can also be coupled to or integrated in (e.g., within the same housing) the user device 170. More generally, the activity tracker 190 can be communicatively coupled with, or physically integrated in (e.g., within a housing), the control system 110, the memory 114, the respiratory therapy system 120, the user device 170, and/or the blood pressure device 180.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for generating physiological data and determining a recommended notification or action for the user according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. As a further example, a fourth alternative system includes the control system 110, the memory device 114, the respiratory therapy system 120, at least one of the one or more sensors 130, the user device 170, and the blood pressure device 180 and/or activity tracker 190. Thus, various systems can be formed using any portion or portions of the components shown and described herein and/or in combination with one or more other components.

As used herein, a sleep session can be defined in a number of ways based on, for example, an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

Generally, the sleep session includes any point in time after the user 210 has laid or sat down in the bed 230 (or another area or object on which they intend to sleep), and has turned on the respiratory therapy device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory therapy device 122, and gets out of bed 230. In some implementations, the sleep session can include additional periods of time, or can be limited to only some of the above-disclosed time periods. For example, the sleep session can be defined to encompass a period of time beginning when the respiratory therapy device 122 begins supplying the pressurized air to the airway or the user 210, ending when the respiratory therapy device 122 stops supplying the pressurized air to the airway of the user 210, and including some or all of the time points in between, when the user 210 is asleep or awake.

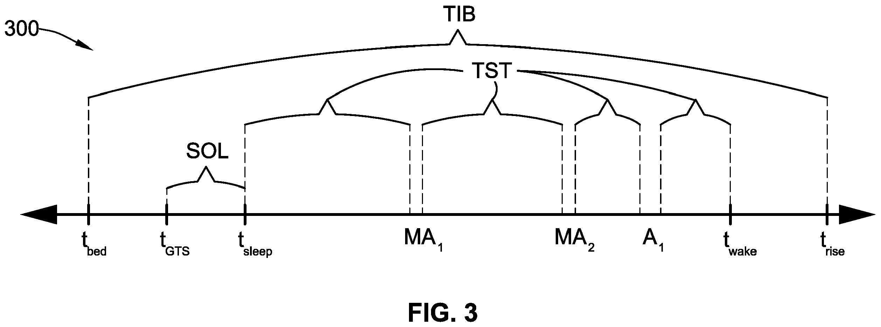

Referring to FIG. 3, an exemplary timeline 300 for a sleep session is illustrated. The timeline 300 includes an enter bed time (t_{bed}), a go-to-sleep time (tors), an initial sleep time (tₛₗₑₑₚ), a first micro-awakening MAi, a second micro-awakening MA₂, an awakening A, a wake-up time (twake), and a rising time (trise).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MAi and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MAi and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (tors) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 300 of FIG. 3, the total sleep time (TST) spans between the initial sleep time tₛₗₑₑₚ and the wake-up time t_{wake}, but excludes the duration of the first micro-awakening MAi, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (tors) and ending at the wake-up time (t_{wake}). In some implementations, a sleep session is defined as starting at the go-to-sleep time (tors) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

Generally, the sleep session includes any point in time after the user 210 has laid or sat down in the bed 230 (or another area or object on which they intend to sleep), and has turned on the respiratory therapy device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory therapy device 122, and gets out of bed 230. In some implementations, the sleep session can include additional periods of time, or can be limited to only some of the above-disclosed time periods. For example, the sleep session can be defined to encompass a period of time beginning when the respiratory therapy device 122 begins supplying the pressurized air to the airway or the user 210, ending when the respiratory therapy device 122 stops supplying the pressurized air to the airway of the user 210, and including some or all of the time points in between, when the user 210 is asleep or awake.

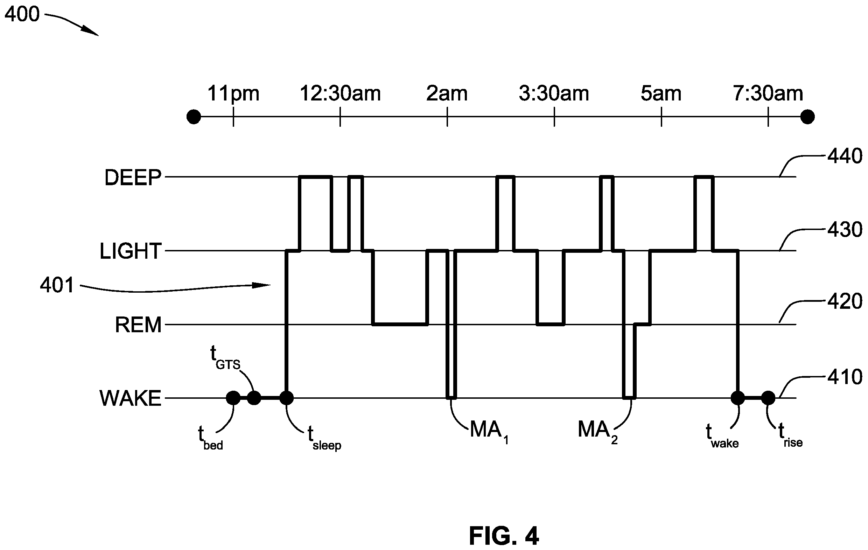

Referring to FIG. 4, an exemplary hypnogram 400 corresponding to the timeline 300 (FIG. 3), according to some implementations, is illustrated. As shown, the hypnogram 400 includes a sleep-wake signal 401, a wakefulness stage axis 410, a REM stage axis 420, a light sleep stage axis 430, and a deep sleep stage axis 440. The intersection between the sleep-wake signal 401 and one of the axes 410-440 is indicative of the sleep stage at any given time during the sleep session.

The sleep-wake signal 401 can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 400 is shown in FIG. 4 as including the light sleep stage axis 430 and the deep sleep stage axis 440, in some implementations, the hypnogram 400 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 400 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (tors) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MAi and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MAi and micro-awakening MA₂ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (tors), the initial sleep time (tₛₗₑₑₚ), one or more first micro-awakenings (e.g., MAi and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (tors), the initial sleep time (tₛₗₑₑₚ), one or more first micro-awakenings (e.g., MAi and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

In some implementations, the system 100 can be used to perform methods for generating one or more custom messages for a user to aid in encouraging a behavioral response. In such implementations, the system 100 generates a custom message based on physiological data and causes the custom message to be communicated to the user, as described in further detail herein. In other implementations, the system 100 can be used to perform methods for selecting one of a plurality of messages to be communicated to the user based on determined base weight values, as described in further detail herein. In still other implementations of the present disclosure, the system 100 can be used to perform methods for determining a total health score for the user, as described in further detail herein. For each of these contemplated methods, the system 100 can cause certain information to be displayed on the display device 172 of the user device 170.

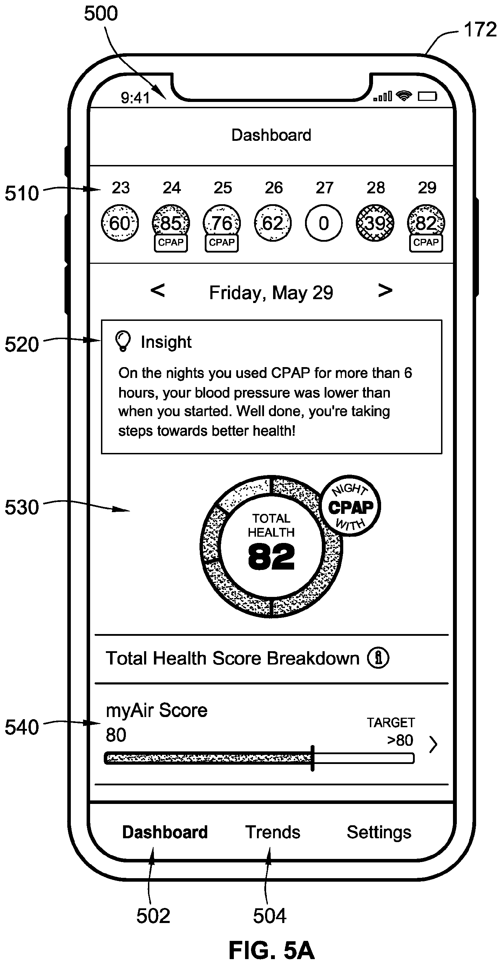

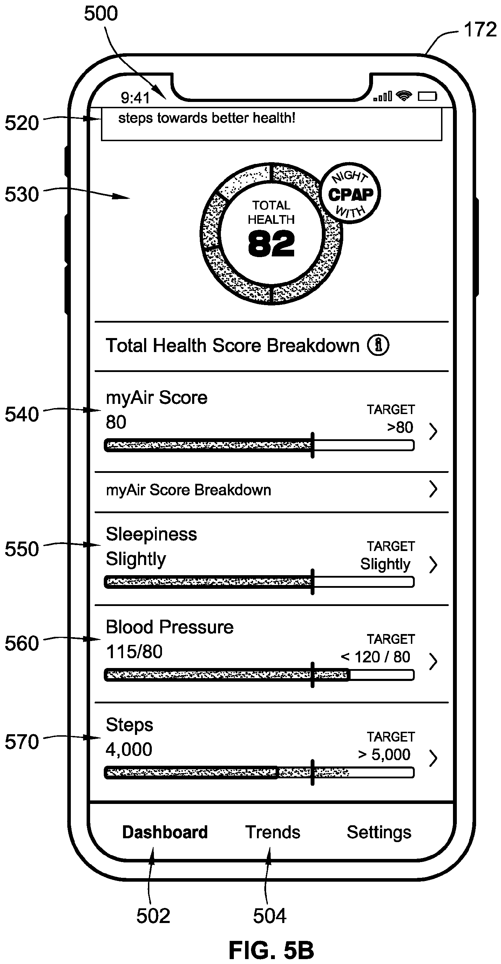

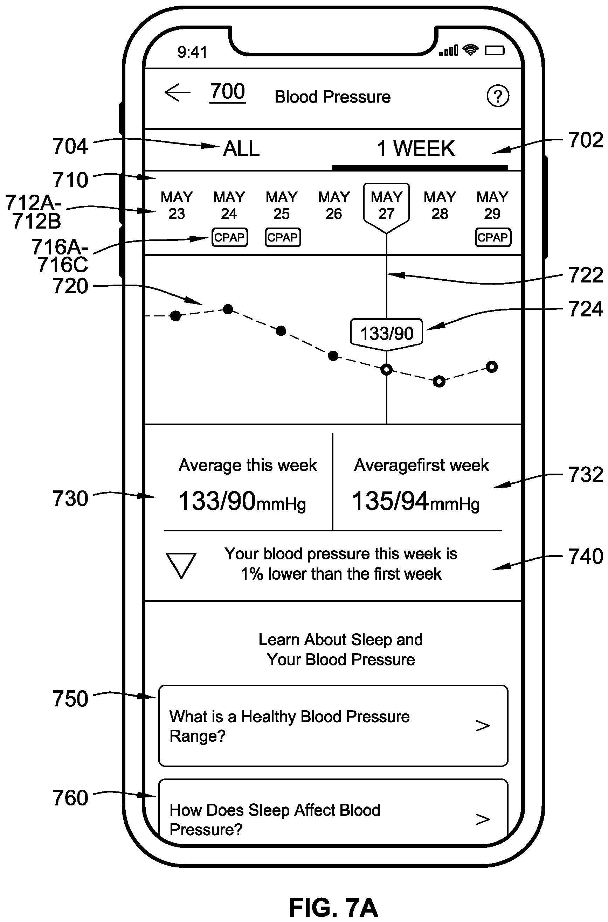

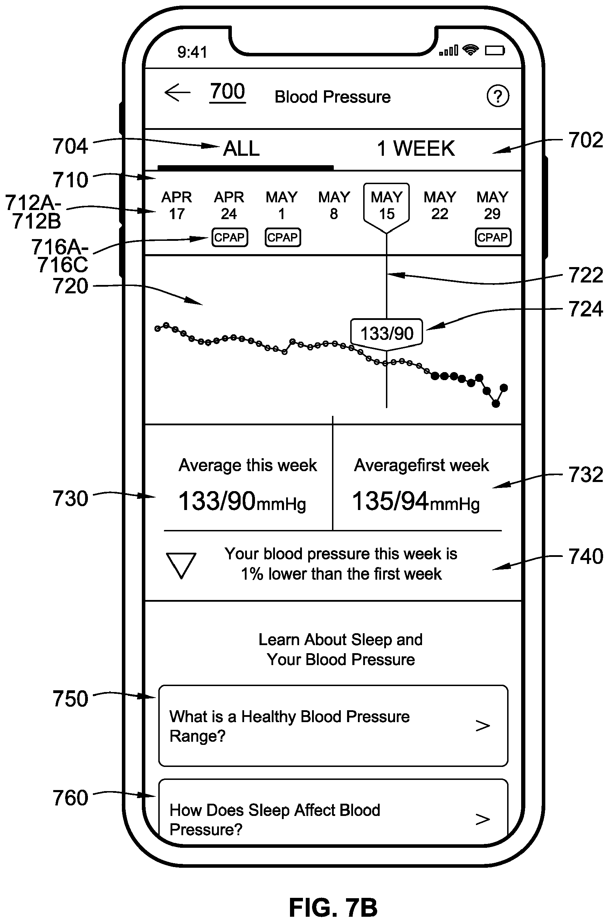

Referring to FIGS. 5A and 5B, a dashboard view 500 is displayed on the display device 172. The dashboard view 500 includes a calendar portion 510 (FIG. 5A), a custom message 520 (FIG. 5A), a total health score indication 530, a sleep score indication 540, a sleepiness score indication 550 (FIG. 5B), a blood pressure measurement indication 560 (FIG. 5B), and an activity measurement indication 570. A dashboard selectable element 502 and a trend selectable element 504 are also displayed on the display device 172 to permit a user to toggle between the dashboard view 500 (FIGS. 5A and 5B) and a trend view 700 (FIGS. 7A and 7B), as described in further detail below.

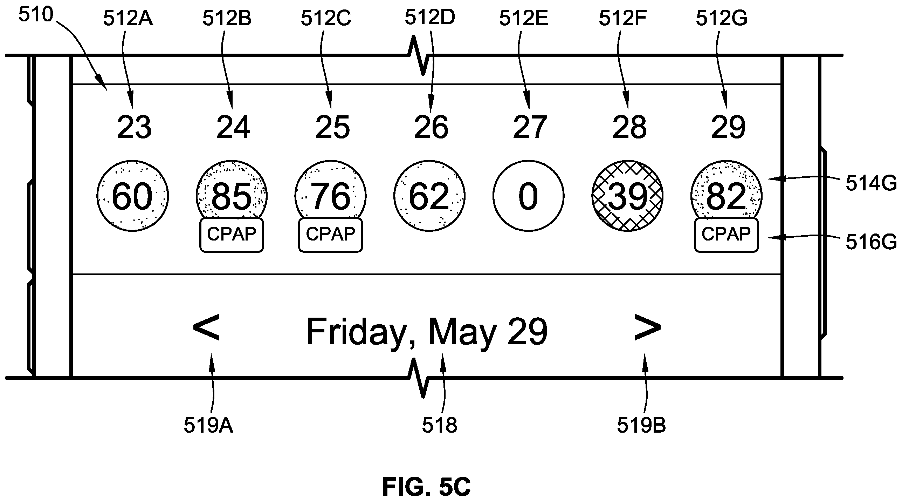

Referring to FIG. 5C, the calendar portion 510 includes a plurality of date indications 512A-512G, a selected date indication 518, a first date navigation element 519A, and a second date navigation element 519B. Each of the plurality of data indications 512A-512G indicates a numerical day during a month, while the selected date indication 516 includes alphanumeric text indicating the selected date and month for which information is displayed in the dashboard view 500. The first date indication 512A corresponds to May 23, the second date indication 512B corresponds to May 24, the third date indication 512C corresponds to May 25, the fourth date indication 512D corresponds to May 26, the fifth date indication 512E corresponds to May 27, the sixth date indication 512F corresponds to May 28, and the seventh date indication 512G corresponds to May 29.

In the example as shown in FIG. 5C, the selected date indication 516 shows that the selected date is Friday, May 29. Each of the custom message 520 (FIG. 5A), the total health score indication 530, the sleep score indication 540, the sleepiness score indication 550 (FIG. 5B), the blood pressure measurement indication 560 (FIG. 5B), and the activity measurement indication 570 described herein correspond to the day reflected by the selected date indication 516 (in this example, Friday, May 29).

Each of the plurality of date indications 512A-512G includes a total health score and optionally a therapy indication. For example, the seventh date indication 512G includes a total health score indication 514G and a therapy indication 516G. The total health score indication 514G includes alphanumeric text indicating the total health score associated with the seventh date indication 512G (in this example, May 29). The total health score indication 514G can also include a color that is based on the relative value of the associated total health score. For example, the total health score indication 514G can be green responsive to the total health score being greater than a first threshold value (e.g., 80), yellow responsive to the total health score being less than the first threshold value (e.g., 80) but greater than a second threshold value (e.g., 50), red responsive to the total health score being less than the second threshold value (e.g., 50) but greater than zero, and gray responsive to the total heath score being zero. Each of the date indications 512A-512F also include a health score indication that is the same as, or similar to, the health score indication 514G for the seventh date indication 512G.

The therapy indication 516G is generally used to indicate whether the user used the respiratory therapy system 120 (FIG. 1) during the corresponding day. As shown, the therapy indication 516G associated with the seventh date indication 512G is partially overlaid on or overlaps the total health score indication 514G and includes alphanumeric text (e.g., "CPAP") indicating that the user used the respiratory therapy system 120 on Friday, May 29. In the example of FIG. 5C, the second date indication 512B and the third date indication 512C also include a therapy indication that is the same as, or similar to, the therapy indication 516G, which indicates that the user used the respiratory therapy system 120 during those corresponding days (in this example, May 24 and May 25). By contrast, in the illustrated example, the first date indictor 512A, the fourth date indicator 512D, the fifth date indicator 512E, and the sixth date indicator 512F do not include a therapy indication. The lack of a therapy indication on or adjacent to the associated total health score indications indicates that the user did not use the respiration therapy system 120 during those days (in this example, May 23 and May 26-28).

Selecting one of the first date navigation element 519A or the second date navigation element 519B changes the selected date. For example, responsive to a selection (e.g., a click or tap) of the first date selection element 519A, the selected date indication 516 is modified to correspond to Thursday, May 28. Responsive to a selection (e.g., a click or tap) of the second date selection element 519B, the selected date indication 516 is modified to correspond to Saturday, May 30. In this manner, any one of the plurality of date indicators 512A-512G can be selected, which in turn updates the custom message 520 (FIG. 5A), the total health score indication 530, the sleep score indication 540, the sleepiness score indication 550 (FIG. 5B), the blood pressure measurement indication 560 (FIG. 5B), and the activity measurement indication 570 to correspond to the selected day.

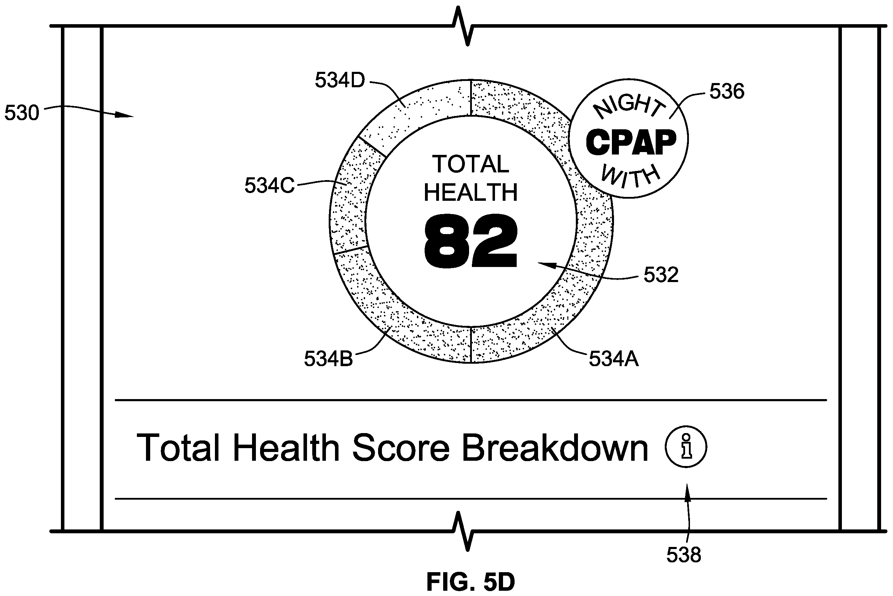

Referring to FIG. 5D, the total health score indication 530 includes a numerical health score indication 532, a plurality of graphical health score component indications 534A-534D, a therapy indication 536, and a selectable information element 538. As described in further detail herein, the total health score for given day is determined as a value between 0 and 100 based at least in part on a determined sleep score, a sleepiness score, a blood pressure measurement, and an activity measurement for that day. However, it will be understood that the total health score may be determined based on more or fewer component scores or measurements. The numerical health score indication 532 indicates the value of the determined total health score for the selected day (e.g., that is between 0 and 100).

The plurality of graphical health score component indications 534A-534D generally represent the relative weight of each of the components used to calculate the total sleep score (e.g., sleep score, sleepiness score, blood pressure measurement, and activity measurement). That is, in the illustrated pie chart format, the relative size of each of the graphical health score component indications 534A-534D corresponds to the relative weight of each component in calculating the total sleep score. For example, the first graphical indication 534A can correspond to a sleep score component, the second graphical indication 534B can correspond to a sleepiness score component, the third graphical indication 534C can correspond to a blood pressure measurement component, and the fourth graphical indication 534D can correspond to an activity level measurement component. For example, the first graphical indication 534A is larger than each of the other graphical indications 534B-534C and represents approximately 50% of the overall pie chart graphic, thus indicating that the first graphical indication 534A is associated with a component of the total health score that has the greatest weight and accounts for approximately 50% of the total health score. In some implementations, each of graphical indications 534A-534D includes a color (e.g., green, yellow, red, or gray) indicating whether the corresponding component is within a healthy threshold range.

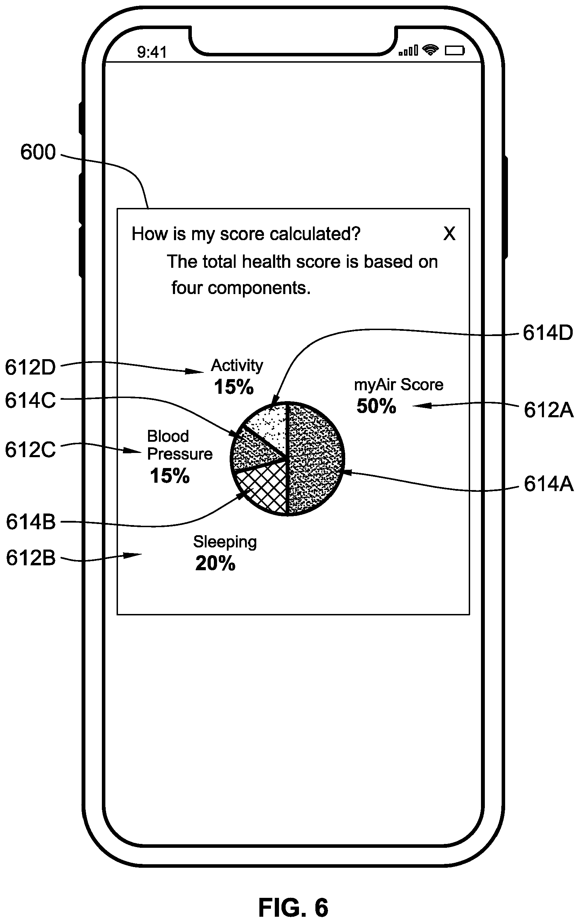

The selectable information element 538 is generally used to navigate from the dashboard view 500 (FIGS. 5A and 5B) to a health score calculation view responsive to a user selection (e.g., a click or tap) of the selectable information element 538. Referring to FIG. 6, responsive to selecting the selectable information element 538 (FIG. 5D), a health score calculation view 600 is displayed on the display device 172. The health score calculation view includes a first indication 610, a plurality of component indications 612A-612D, and a plurality of graphical component indications 614A-614D.

The first indication 610 generally includes alphanumeric text describing how the total health score is calculated (e.g., "your total health score is based on your myAir score, your reported sleepiness, your blood pressure, and your activity").

The plurality of graphical component indications 614A-614D are the same as, or similar to, the plurality of graphical health score component indications 534A-534D (FIG. 5D). The plurality of component indications 612A-612D generally describe both the component of the total health score corresponding to each of the plurality of graphical component indications 614A-614D and the weight associated with that component. For example, the first component indication 612A indicates that the first graphical component indication 614A is a sleep score and accounts for 50% of the total health score. The second component indication 612B indicates that the second graphical component indication 614B is a sleepiness score and accounts for 20% of the total health score. The third component indication 612C indicates that the third graphical component indication 614C is a blood pressure measurement and accounts for 15% of the total health score. The fourth component indication 612D indicates that the fourth graphical component indication 614D is an activity measurement and accounts for 15% of the total health score.

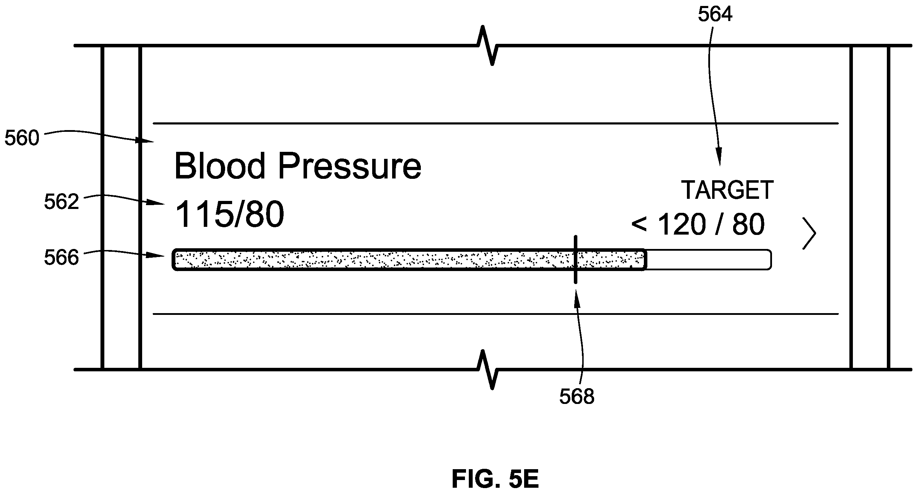

Referring back to FIGS. 5A and 5B, the dashboard view 500 also includes the sleep score indication 540, the sleepiness score indication 550 (FIG. 5B), the blood pressure measurement indication 560 (FIG. 5B), and the activity measurement indication 570. Referring to FIG. 5E, the blood pressure measurement indication 560 includes a numerical measurement indication 562, a numerical target indication 564, a graphical measurement indication 566, and a graphical target indication 568. The numerical measurement indication 562 indicates the blood pressure measurement in alphanumeric text (e.g., a systolic value of 115 mm Hg and a diastolic value of 80 mm Hg), and can include a systolic value, a diastolic value, or both. The numerical target indication 564 indicates a target blood pressure measurement(s) in alphanumeric text (e.g., a target systolic value less than 120 mm Hg and a diastolic value less than 80 mm Hg). As described in further detail herein, the target blood pressure can be determined based on a predetermined value (e.g., from the American Heart Association) or customized for the particular user (e.g., based on demographic and/or medical information associated with the user), and can include a target systolic value, a target diastolic value, or both.

The graphical measurement indication 566 and the graphical target 568 generally correspond to the numerical measurement indication 562 and numerical target indication 564, respectively, and display the values graphically or visually rather than using alphanumeric text. As shown, the graphical target indication 568 can be a vertical line or tick mark, and the graphical measurement indication 566 can be a horizontal line. In the illustrated example, the graphical measurement indication 566 extending to the right of the graphical target indication 568 indicates that the measured blood pressure meets or exceeds the target blood pressure. Conversely, the graphical measurement indication 566 stopping to the left of the graphical target indication 568 indicates that the measured blood pressure fails to meet the target blood pressure. The graphical measurement indication 566 and graphical target indication 568 can include a color that further indicates whether the target has been achieved (e.g., green if the target is met, yellow if the target is not met, or red if the measurement is well short of the target or at an unhealthy value). Thus, graphical measurement indication 566 and the graphical target 568 generally convey the same information as the numerical measurement indication 562 and numerical target indication 564, but in a visual format that may be easier or more intuitive for the user to understand.

Each of the sleep score indication 540, the sleepiness score indication 550 (FIG. 5B), and the activity measurement indication 570 can include a numerical measurement indication, a numerical target indication, a graphical measurement indication, and a graphical target indication that are the same as, or similar to, the numerical measurement indication 562, the numerical target indication 564, the graphical measurement indication 566, and the graphical target indication 568, respectively, of the blood pressure measurement indication 560 (FIG. 5E).

In some implementations, all or a portion of the blood pressure measurement indication 560 (FIGS. 5B and 5E) is selectable by the user (e.g., responsive to a click or tap) such that the user can navigate to a blood pressure view. Referring generally to FIGS. 7A and 7B, in such implementations, a blood pressure view 700 is displayed on the display device 172 responsive to a selection of the blood pressure measurement indication 560 (FIGS. 5B and 5E). The blood pressure view 700 includes a first time filter 702, a second time filter 704, a calendar portion 710, a graphical trend indication 720, a current week average indication 730, a first week average indication 732, a trend indication 740, a first selectable information element 750, and a second selectable information element 760.

The calendar portion 710 of the blood pressure view 700 is similar to the calendar view 510 (FIG. 5C) described herein in that the calendar portion 710 includes a plurality of date indications and optionally includes one or more therapy indications that are the same as, or similar to the plurality of date indications 512A-512G and therapy indication 512G described above. The first time filter 702 and the second time filter 704 can be selected by the user (e.g., responsive to a click or tap) to specify the number of days included in the calendar portion 710.

In FIG. 7A, the first time filter 702 is selected. In this example, the first time filter 702 is associated with one week (7 days). Thus, in this particular example, the calendar portion 710 includes a plurality of date indications 712A-712G, each of which corresponds to one of the days in the week (May 23 to May 29). The second date indication 712B (corresponding to May 24) includes a therapy indication 714B, the third date indication 712C (corresponding to May 25) includes a therapy indication 714C, and the seventh date indication 712G (corresponding to May 29) includes a therapy indication 714G, which indicate that the user used the respiratory therapy system 120 (FIG. 1) during those days, but did not do so during the other days (May 23 and 26-28).

Referring to FIG. 7B, the first time filter 702 is selected. In this example, the second time filter 704 is associated with all days in which data was recorded. Thus, in this particular example, the plurality of date indications 712A-712G correspond to a range of dates that is greater than one week (FIG. 7A). The time period in the example shown in FIG. 7B extends between April 17 and May 29 (i.e., 8 weeks), and the date indications 712A-712G are interpolated between the start date (April 17) and the end date (May 29). The first date indication 712A correspond to April 17, the second date indication 712B corresponds to April 24, the third date indication 712C corresponds to May 1, the fourth date indication 712D corresponds to May 8, the fifth date indication 712E corresponds to May 15, the sixth date indication 712F corresponds to May 22, and the seventh date indication 712G corresponds to May 29. The therapy indication 714B corresponds to April 24, therapy indication 714C corresponds to May 1, and therapy indication 714G corresponds to May 29.

Referring back to FIG. 7A, the graphical trend indication 720 generally depicts a trend in the blood pressure measurement throughout the selected time period (in the example of FIG. 7A, one week). The graphical trend indication 720 includes a plurality of data points, each of which corresponds to one of the plurality of date indications 712A-712G. Each of the plurality of date indications is selectable by the user (e.g., responsive to a click or tap) to display additional information associated with the blood pressure measurement for that day. In FIG. 7A, the fifth date indication 712E (May 27) is selected, which causes a visual identifier 722 and a blood pressure measurement indicator 724 to be to be at least partially overlaid on the graphical trend indication 720. The visual identifier 722 extends from the date indication 712E to a corresponding one of the data points to clearly identify that data point as corresponding to the selected date (May 27). The blood pressure measurement indication 724 indicates the associated determined blood pressure measurement for the selected date (e.g., for May 27, the blood pressure measurement for the user was 133 / 90).

Referring to FIG. 7B, the graphical trend indication 720 is modified responsive to a selection of the second date filter 704 so that the graphical trend indication 720 includes a data point for each day within the selected time period (in this example, each day between April 17 and May 29). As shown in FIG. 7B, selecting a different one of the date indications also modifies the visual identifier 722 and the blood pressure measurement indicator 724 to correspond to the selected date.

The current week average indication 730 indicates an average blood pressure measurement for the current week (in the example of FIG. 7A, 133/90 mm Hg). The average blood pressure measurement is determined by adding the blood measurements associated with each of the date indications 712A-712G and dividing by the number of days (in this example, 7 days). The first week average indication 732 indicates an average blood pressure measurement for the first week during which data associated with the user is collected.

The textual trend indication 740 generally provides information describing a determined trend for the blood pressure measurement during a time period. For example, the trend indication 740 can indicate that a trend in the average blood pressure measurement for the current week (indicated by the current week average indication 730) relative to the average blood pressure measurement for the first week (indicated by the first week average indication 732). For example, the trend indication 740 can include alphanumeric text (e.g., "Your blood pressure this week is 1% lower than the first week") and/or one or more symbols (e.g., a down arrow indicating that the blood pressure is trending down).

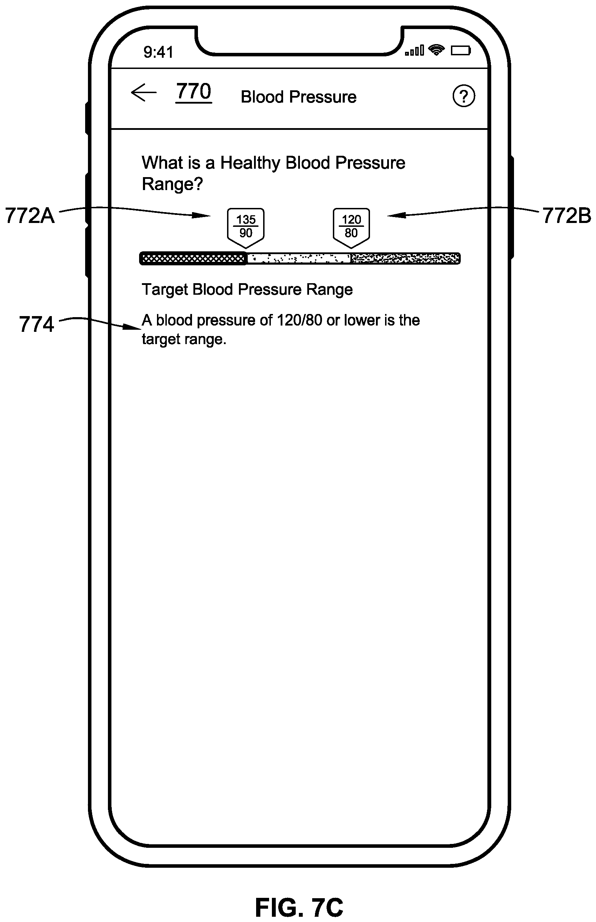

The first selectable information element 750 is selectable by a user (e.g., by clicking or tapping) so that the user can navigate to a display providing further information about the blood pressure measurements. For example, referring to FIG. 7C, selecting selectable information element 750 (FIGS. 7A and 7B) causes a blood pressure target view 770 to be displayed on the display device 172. The blood pressure target view 770 includes a first target threshold indication 772A, a second target threshold indication 772B, and a textual indication 774. The first target indication 772A includes an upper threshold for the blood pressure measurement and the second target indication 772B indicates a lower threshold for the blood pressure measurement. For example, a blood pressure measurement that is at or below the lower threshold is considered in the targeted range (e.g., healthy), a blood pressure measurement that is between the lower threshold and the upper threshold is considered neutral, and a blood pressure measurement that is greater than the upper threshold is generally considered unhealthy.

The upper and lower blood pressure target values can be selected using a predetermined range of values (e.g., values specified by the American Heart Association or a medical provider). Alternatively, the upper and lower blood pressure target values can be customized for the user based at least in part on a user profile associated with the user (e.g., demographic information, medical information, etc.). In some implementations, the upper and lower blood pressure target values are determined using a trained machine learning algorithm.

The second selectable information element 760 is similar to the first selectable information element 750 in that the second selectable information element 760 is selectable by the user (e.g., via a click or tap) and causes information describing a correlation between the blood pressure measurements and sleep to be displayed on the display device 172.

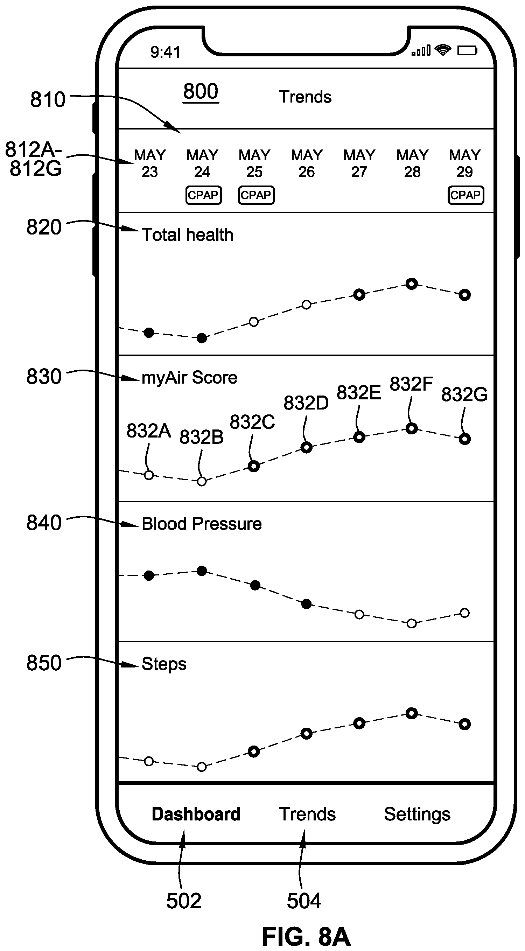

Referring to FIG. 8A, responsive to a selection (e.g., click or tap) of the trend selectable element 504, a trend view 800 is caused to be displayed on the display device 172. The trend view can include a calendar portion 810, a total health score trend 820, a sleep score trend 830, a blood pressure measurement trend 840, and an activity level measurement trend 850.

The calendar portion 810 is the same as, or similar to, the calendar portion 510 (FIG. 5C) described above and includes a plurality of date indications 812A-812G (e.g., corresponding to May 23 - May 29). Each of the total health score trend 820, the sleep score trend 830, the blood pressure measurement trend 840, and the activity level measurement trend 850 are generally used to indicate a trend in the associated measurement for the time period included in the calendar portion 810 (e.g., 3 days, 1 week, 2 weeks, 1 month, 2 months, 6 months, 1 year, etc.).

Each of the total health score trend 820, the sleep score trend 830, the blood pressure measurement trend 840, and the activity level measurement trend 850 include a plurality of data points, where each of the plurality of data points corresponds to an associated measurement for the corresponding date. For example, the sleep score trend 830 includes a plurality of data points 832A-832G corresponding to the plurality of date indications 812A-812G in the calendar portion 810. As shown, the sleep score trend 830 can also include a dashed line extending between adjacent ones of the plurality of data points 832A-832G. Further, each of the plurality of data points 832A-832G can include a color indicative of whether the associated measurement is within a health range (e.g., green is indicative of a healthy measurement, yellow is indicative of a neutral measurement, red is indicative of an unhealthy measurement, etc.).

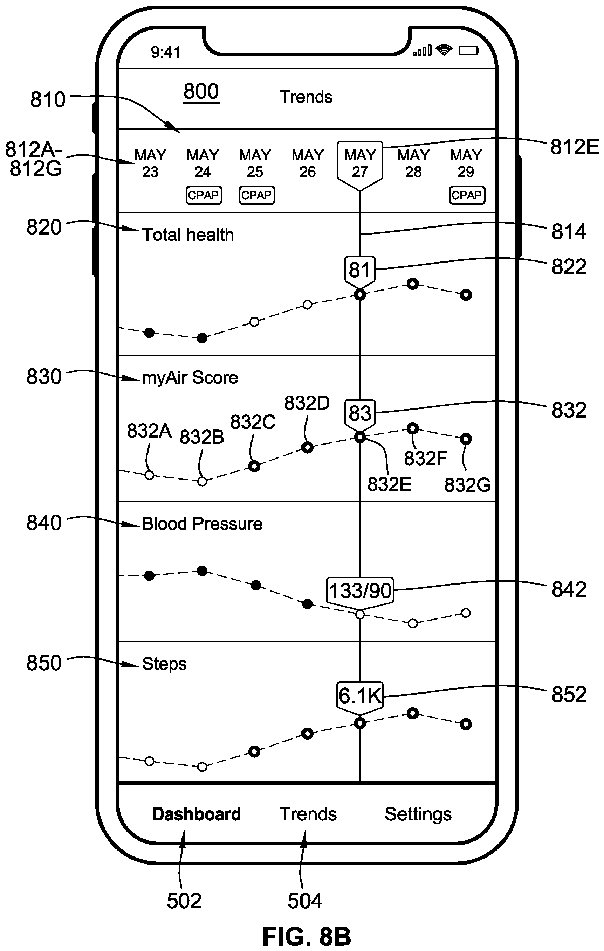

Referring to FIG. 8B, in some implementations, selecting one of the date indications 812A-812G causes a visual identifier 814 to be displayed. The visual identifier 814 extends from the selected one of the date indications 812A-812G (in the example of FIG. 8B, May 27) through the corresponding data points in the total health score trend 820, the sleep score trend 830, the blood pressure measurement trend 840, and the activity level measurement trend 850. Additionally, the total health score trend 820 can include a measurement indicator 822 indicating the determined total sleep score for the selected day (e.g., 81), the sleep score trend 830 can include a measurement indicator 832 indicating the determined sleep score for the selected day (e.g., 83), the blood pressure measurement trend 840 can include a measurement indicator 842 indicating the measured blood pressure for the selected day (e.g., 133/90), and the activity level measurement trend 850 can include a measurement indicator 852 indicating the measured activity level for the selected day (e.g., 6,100 steps).

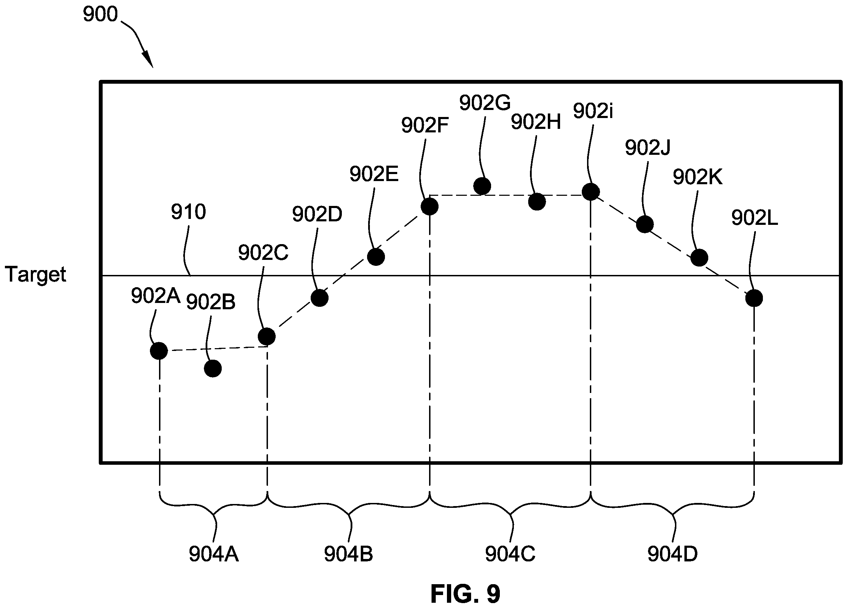

In some implementations, the plurality of data points associated with each of the total health score trend 820, the sleep score trend 830, the blood pressure measurement trend 840, and the activity level measurement trend 850 can be used to determine a trend indication for a time period. Referring to FIG. 9, an exemplary trend 900 is illustrated. The trend 900 can correspond to any of the sleep-related or non-sleep-related parameters described herein (e.g., a sleep score, a sleepiness core, a blood pressure measurement, or an activity level measurement). The trend 900 includes a plurality of data points 902A-902L. Each data point 902A-902L corresponds to a different day. Thus, in this example, the trend 900 includes 12 days.

One or more trend indications can be determined using all or some of the data points 902A-902L. The trend indication can be a positive or good trend indication, a negative or bad trend indication, a stable-good trend indication, or a stable-bad trend indication. These trend indications can be determined based at least in part on a slope of a fitted line for a given time period. A positive or good trend indication generally indicates that the values associated with parameter, during a given time period, are trending in a desired direction. For example, the sleep score parameter, the trend indication is a positive or good trend indication in response to a determination that the slope of the fitted line is greater than a first threshold value (e.g., greater than about 0.05). In another example, for the blood pressure measurement parameter, the trend indication is a positive trend indication in response to a determination that the slope of the fitted line is less than a first threshold value (e.g., less than about -0.05). In other words, the sign (positive or negative) of the determined slope can be inverted based on the type of parameter. Conversely, a negative trend indication generally indicates that the values associated with parameter, during a given time period, are trending away from the desired direction. For example, for the sleep score parameter, the trend indication is a negative trend indication in response to a determination that the slope of the fitted line is less than a second threshold value (e.g., less than about -0.05). As another example, for the blood pressure measurement parameter, the trend indication is a negative trend indication in response to a determination that the slope of the fitted line is greater than a second threshold value (e.g., greater than about 0.05).

A trend indication can be stable responsive to the measured values being within a range of slope threshold values such that the slope of the fitted line is between a lower slope threshold value and an upper slope threshold value (e.g., between about -0.05 and about 0.05). The trend indication can be stable-good or stable-bad based at least in part on the range of healthy threshold values described herein. For example, if an average of the first value and the second value for one of the parameters is within the range of healthy threshold ranges for that parameter, and the slope of the fitted line is between the upper and lower slope threshold values, the trend indication is a stable-good indication. Conversely, if an average of the first value and the second value for one of the parameters is not within the range of healthy threshold ranges for that parameter, and the slope of the fitted line is between the upper and lower slope threshold values, the trend indication is a stable-bad indication.

Alternatively, the stable trend indication can be stable-good responsive to each of the first value and the second value being within the range of healthy values. Similarly, the stable trend indication can be stable-bad responsive to each of the first value and the second value being outside the range of healthy values.

Alternatively, the stable trend indication can be stable-good responsive at least one of the first value and the second value being within the range of healthy values. Similarly, the stable trend indication can be stable-bad responsive to at least one of the first value and the second value being outside the range of healthy values.

While the range of slope threshold values has been described herein as being between about -0.05 and about 0.05, more generally, the upper and lower slope threshold values can be any suitable number (e.g., between -0.01 and about 0.01, between about -0.1 and about 0.1, between about -0.3 and about 0.3, etc.).

For example, a first line 904A can be fitted between data points 902A-902C, which correspond to a first time period. The first line 904A has a slope that is approximately zero and between about -0.05 and about 0.05. Further, each of the data points 902A-902C associated with the first line 904A is below a target threshold value 910. Thus, for the first time period between data point 902A and data point 902C, the trend is a stable-bad trend.

As another example, a second line 904B can be fitted between data points 902D-902F, which correspond to a second time period. The second line 904B has a slope that is greater than about 0.05. Thus, for the second time period between data point 902D and data point 902F, the trend is a positive trend.

As a further example, a third line 904C can be fitted between data points 902F-902I, which correspond to a third time period. The third line 904C has a slope is approximately zero and between about -0.05 and about 0.05. Further, each of the data points 902F-902L associated with the third line 904C is above the target threshold value 910. Thus, for the third time period between data points 902F-902I, the trend is a stable-good trend.

As another example, a fourth line 904D can be fitted between data points 902I-902L, which correspond to a fourth time period. The second line 904D has a slope that is less than about 0.05. Thus, for the fourth time period between data point 902I and data point 902L, the trend is a negative trend.

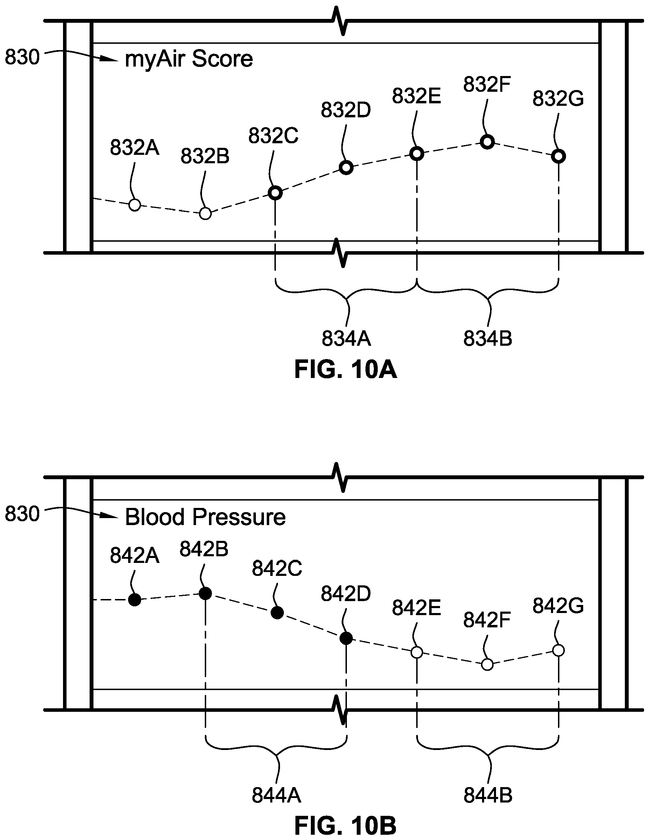

Referring to FIG. 10A, the sleep score trend 830 includes a plurality of data points 832A-832G. One or more trend indications can be determined based on the data points 832A-832G (e.g., by fitting lines between two or more data points). For example, a first line 834A can be fitted between data point 832C and data point 832E. As shown, the first line 834A has a slope that is greater than about 0.05. Thus, the trend indication for this associated time period (e.g., between May 25 and May 27, as shown in FIG. 8A), is a positive trend. As another example, a second line 834B can be fitted between data point 832E and data point 832G. The second line 834B has a slope that is between about -0.05 and about 0.05. Further, the measurements corresponding to each of the data points 832A-832G is greater than a predetermined threshold value for the determined sleep score. Thus, the trend indication for this associated time period (e.g., between May 27 and May 29, as shown in FIG. 8A), is a stable-good trend.

Referring to FIG. 10B, the blood pressure measurement trend 840 includes a plurality of data points 842A-842G. One or more trend indications can be determined based on the data points 842A-842G (e.g., by fitting lines between two or more data points). For example, a first line 844A can be fitted between data point 842B and data point 842E. As shown, the first line 844A has a slope that is less than about -0.05. Thus, the trend indication for this associated time period (e.g., between May 24 and May 26, as shown in FIG. 8A), is a negative trend. As another example, a second line 844B can be fitted between data point 842E and data point 842G. The second line 844B has a slope that is between about -0.05 and about 0.05. Further, the measurements corresponding to each of the data points 842A-842G is greater than a predetermined threshold value for the blood pressure measurement. Thus, the trend indication for this associated time period (e.g., between May 27 and May 29, as shown in FIG. 8A), is a stable-bad trend.

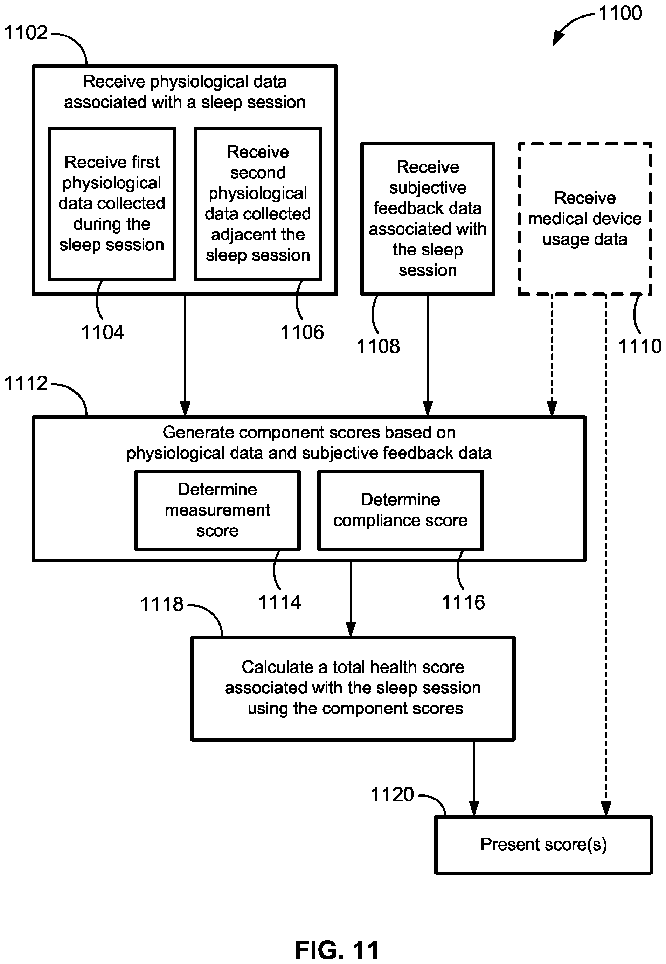

FIG. 11 is a flowchart depicting a process 1100 for determining a health score, according to certain aspects of the present disclosure. Process 1100 can be performed by any suitable computing device(s), such as any device(s) of system 100 of FIG. 1. In some cases, process 1100 can be performed by a smartphone, tablet, home computer, or other such device.

At block 1102, physiological data associated with a sleep session can be received. Receiving physiological data can include receiving sensor data from one or more sensors (e.g., one or more sensors 130, blood pressure device 180, or activity tracker 190 of FIG. 1). The one or more sensors can be housed in and/or physically coupled to the device(s) performing process 1100, or can be separate. Receiving physiological data at block 1102 can occur over the course of any suitable duration. Physiological data associated with a sleep session can comprise physiological data collected during the sleep session, as well as physiological data collected within a duration of time adjacent a sleep session (e.g., before and/or after the given sleep session).

Receiving physiological data at block 1102 can include receiving first physiological data collected during the sleep session at block 1104 and receiving second physiological data collecting adjacent the sleep session at block 1106. While second physiological data can be received after first physiological data, that need not always be the case.

As described herein, the sleep session can be defined in a number of ways based on, for example, an initial start time and an end time, although any suitable definition for a sleep session can be used. A duration adjacent a sleep session can include a period of time before the sleep session and/or a period of time after the sleep session. In some cases, a duration adjacent a sleep session can include a predetermined amount of time before the sleep session (e.g., 30 minutes prior to the sleep session) or can include a duration of time between the end of a previous sleep session and a start of the current sleep session. In some cases, a duration adjacent a sleep session can include a predetermined amount of time after the sleep session (e.g., 16 hours after the end of the sleep session) or can include a duration of time between the end of the current sleep session and the start of a subsequent sleep session. In some cases, a previous or subsequent sleep session can be defined as a previous or subsequent sleep session that is at least a predetermined amount of time before or after, respectively, the current sleep session. For example, if the current sleep session ends at 6:00 AM and a 12-hour delay is set before another sleep session can be counted as a subsequent sleep session, an additional sleep session taken at 2:00 PM (e.g., an afternoon nap) may be ignored, and the subsequent sleep session may be a next sleep session that starts at 10:00 PM. Other techniques can be used to define the duration of time adjacent the sleep session.

Receiving the first physiological data at block 1104 can include receiving physiological data that is collected during the sleep session. Examples of such physiological data can be data associated with breathing rate while sleeping, heart rate while sleeping, sleep duration, and the like. Other physiological data as disclosed herein can be collected during a sleep session at block 1104.

Receiving physiological data at block 1106 can include receiving physiological data that is collected during a duration of time adjacent the sleep session. Examples of such physiological data can be data associated with blood pressure, physical activity (e.g., step counts), heart rate information, and the like. Other physiological data as disclosed herein can be collected during a duration of time adjacent a sleep session at block 1106. In some cases, physiological data collected at block 1106 can include measurement information (e.g., an actual blood pressure measurement, such as 120 mmHg systolic over 80 mmHg diastolic) as well as compliance information (e.g., information associated with the act of taking and/or recording the blood pressure measurement). For example, physiological data collected at block 1106 can include three different blood pressure measurements for a user over the course of a duration adjacent a sleep session. The actual values from these measurements may be considered measurement information, whereas the number of times the user made these measurements (e.g., three times) can be considered compliance information. In some cases, compliance information can include whether or not an action (e.g., measurement) was taken, a number of times an action was taken, a time of day an action was taken, a duration of time for which measurements were taken, or any other metadata associated with the measurement (e.g., metadata associated with the measurement and not the actual measurement value itself).

At block 1108, subjective feedback data associated with the sleep session can be received. Subjective feedback data can include information about a perceived rating or measurement associated with the sleep session. In an example, subjective feedback data can include an indication of sleepiness (e.g., very tired, tired, moderately awake, awake, or very awake) as provided by the user or someone considering the user. In some cases, receiving subjective feedback data at block 1108 can occur in response to transmitting a request for subjective feedback, such as providing a prompt on a user's computing device to provide the subjective feedback. In some cases, subjective feedback data can include measurement information (e.g., a value associated with the subjective feedback) and compliance information (e.g., a value associated with whether or not subjective feedback was given).

In some cases, physiological data received at block 1102 can be used to suggest a potential subjective feedback measurement, which when confirmed or modified, can be used as the subjective feedback data. For example, if the physiological data received at block 1102 is indicative of poor sleep, the system may prompt the user for subjective feedback and default the selection options to "tired" until the user makes a change to the subjective feedback selection. By contrast, if the physiological data received at block 1102 is indicative of strong sleep, the system may prompt the user for subjective feedback and default the selection options to "very awake" until the user makes a change to the subjective feedback selection.

In some cases, the system can generate estimated subjective feedback data based on physiological data received at block 1102. For example, receiving physiological data associated with user response times to stimuli (e.g., response time between a given stimulus and a user action identifiable from the physiological data, such as a recognizable breathing action) can be used to estimate a degree of sleepiness. In some cases, the system can use subjective feedback received at block 1108 to train a model or algorithm for determining estimated subjective feedback data based on physiological data received at block 1102.

Estimated subjective feedback data can be used in lieu of subjective feedback data, such as when subjective feedback data is not received (e.g., a user forgets to provide subjective feedback data) or desired (e.g., the system is set up to use only estimated subjective feedback data instead of user-provided subjective feedback data). Thus, in some cases, receiving subjective feedback data at block 1108 can be replaced by receiving estimated subjective feedback data based on the received physiological data at block 1102 (e.g., by applying the received physiological data to a model or algorithm trained using historical subjective feedback data and corresponding historical physiological data).

At block 1112, component scores can be generated based on physiological data and subjective feedback data. The set of component scores generated at block 1112 can include at least 1, 2, 3, 4, or more component scores. In some cases, the set of component scores includes a sleep score, a subjective sleepiness score, a blood pressure score, an activity score, or any combination thereof. In some cases, other or additional component scores can be used. Each component score is associated with a particular component that is evaluated for purposes of the total health score. For example, the sleep score can be associated with a sleep component (e.g., an objective during-sleep sleep quality component), the sleepiness score can be associated with a sleepiness component (e.g., a subjective post-sleep sleep quality component), the blood pressure score can be associated with a blood pressure component, and the activity score can be associated with an activity component (e.g., a step count component). Other or additional component scores can be used to evaluate other or additional components.

Generating the component scores at block 1112 includes generating a component score for each component of the set of components. Each component score can be generated based on the physiological data received at block 1102 and the subjective feedback data at block 1108. Each component score is thus associated with a particular sleep session (e.g., the sleep session associated with the physiological data received at block 1102 and the subjective feedback data at block 1108). Component scores can be generated based on any suitable technique, dependent on the form of the data.

In some cases, generating a component score includes determining a measurement score at block 1114. A measurement score is a score that is based on the measured value associated with a component (e.g., the actual blood pressure values of a blood pressure measurement). Generating a measurement score at block 1114 can include applying received data (e.g., received physiological data or received subjective feedback data) to a formula or algorithm. In some cases, received data can be binned, with measurement scores assigned to each bin. For example, a sleepiness measurement score of 5 can be assigned to subjective feedback data associated with a sleepiness component that is "very awake." In another example, an activity score of 2 can be assigned to received physiological data associated with an activity component that indicates between 15% and 25% of a daily number of target steps was achieved.

In some cases, a measurement score can be established based on one or more targets (e.g., one or more target values or one or more target ranges). For example, if it is desirable (e.g., more healthy) to achieve a sleep score at or greater than 80, the target value may be set at 80 and measurement scores for a sleep score that are below 80 may be lower than measurement scores for a sleep score that exceed 80. In another example, if it is desirable to achieve a blood pressure (e.g., systolic, diastolic, or a combination thereof) within a given range or ranges, higher measurement scores can be associated with measurement values within the given range or ranges. Thus, measurement scores can be based on targets for each particular component (e.g., based on analyzing measured values in light of targets for each particular component). These targets can be preset and/or automatically adjusted. In some cases, historical total health scores and/or historical component scores can be used to modify or adjust targets. In some cases, supplemental information can be included to generate a component score(s), such as demographic information associated with the user, medical history information associated with the user, or family health information associated with the user.

In some cases, generating a component score includes determining a compliance score at block 1116. A compliance score is a score that is based on metadata associated with a measurement that is associated with a component (e.g., the number of times blood pressure measurements were taken). A compliance score can be based on whether or not a particular measurement was taken and/or particular data was received. A compliance score can be based on a number of measurement instances or a duration of measurement associate with the one or more measurements used to generate a measurement score. Generating a compliance score at block 1116 can include applying received data (e.g., received physiological data or received subjective feedback data) to a formula or algorithm. In some cases, received data can be binned, with compliance scores assigned to each bin. For example, a blood pressure compliance score of 5 can be assigned to received physiological data associated with a blood pressure component that indicates blood pressure measurements were taken twice during the duration adjacent the sleep session.

In some cases, a component score is a measurement score, a compliance score, or a score calculated from a combination thereof (e.g., a sum of a measurement score and a compliance score). In some cases, not all component scores in the set of component scores needs to make use of compliance scores.

In an example, a component score can be a sleep score. The sleep score can be based on a combination of factors related to a sleep session. The sleep score can be associated with a therapy, such as a therapy applied using a respiratory system (e.g., respiratory therapy system 120 of FIG. 1). In some cases, the sleep score is based on a usage score, an interface score, an interruption score, an event score, or any combination thereof. The usage score can be a score based on a duration of time therapy was applied (e.g., a duration of time the respiratory system was used). The interface score can be an indication of effectiveness of a user interface seal (e.g., the seal of user interface 124 of respiratory therapy system 120 of FIG. 1). The interface score can be based on a measurement of a seal efficiency. The interruption score can be based on a number of times the user removes and/or replaces the user interface during a sleep session. The event score can be based on a number of events (e.g., snoring, an apnea, a hypopnea, a restless leg, a sleeping disorder, choking, an increased heart rate, labored breathing, an asthma attack, an epileptic episode, a seizure, or any combination thereof) that occur during the sleep session, or an average of such events per predetermined unit of time (e.g., events per hour).

In an example, the usage score accounts for 70% of the sleep score, the interface score accounts for 20% of the sleep score, the interruption score accounts for 5% of the sleep score, and the event score accounts for 5% of the sleep score. In some cases, these percentages can be adjusted, such as plus or minus 1%, 2%, 3%, 4%, 5%, or more. In some cases, other factors can be used to generate a sleep score.

The various scores (e.g., usage score, interface score, interruption score, and event score) that make up the sleep score can be determined or calculated from physiological data from block 1102 (e.g., first physiological data from block 1104) and/or received medical device usage data from block 1110.

In an example, a component score can be a sleepiness score. The sleepiness score can be based on subjective feedback data from block 1108. The sleepiness score can be based at least in part on a sleepiness measurement score, a sleepiness compliance score, or a combination thereof. In an example, subjective feedback associated with a sleepiness score can take the form of five different options (e.g., very tired, tired, moderately awake, awake, and very awake), each associated with a point value (e.g., 0, 1, 2, 3, and 5, respectively). Thus, the sleepiness measurement score can be the point value associated with whichever option the user selected when providing subjective feedback. Other points values or calculations can be used. The sleepiness compliance score, if used, can provide points based on whether or not the subjective feedback was provided. For example, if no subjective feedback is provided, the sleepiness compliance score may be a first number (e.g., 0), but if subjective feedback is provided, the sleepiness compliance score may be a second number (e.g., 5). Other durations and point values may be used, as well as other measures of compliance. In some cases, the sleepiness score is a combination of the sleepiness measurement score and the sleepiness compliance score. In such an example, a user who provides subjective feedback of "moderately awake" may receive a sleepiness measurement score of 2 and a sleepiness compliance score of 5, for a total sleepiness score of 7.

In an example, a component score can be a blood pressure score. The blood pressure score can be based on physiological data from block 1102. The blood pressure score can be based at least in part on a blood pressure measurement score, a blood pressure compliance score, or a combination thereof. In an example, physiological data associated with blood pressure can be received, such as from a wearable device (e.g., blood pressure cuff) or from user input (e.g., fields on a user input interface). Point values can be attributed to different bins based on targets for blood pressure. In an example, a first bin valued at 0 points can be associated with a systolic measurement at or greater than 140 mmHg, a second bin valued at 3 points can be associated with a systolic measurement between 130 mmHg and 139 mmHg, a third bin valued at 8 points can be associated with a systolic measurement between 120 mmHg and 129 mmHg, and a fourth bin valued at 10 points can be associated with a systolic measurement below 120 mmHg. Any number of bins can be used, with any number of associated point values and targets. In some cases, a blood pressure score is based solely off systolic pressure measurements. In some cases, a blood pressure score is based on diastolic pressure measurements or a combination of systolic and diastolic pressure measurements. For example, a first bin valued at 0 points can be associated with a systolic measurement at or greater than 140 mmHg or a diastolic measurement at or greater than 90 mmHg, a second bin valued at 3 points can be associated with a systolic measurement between 130 mmHg and 139 mmHg or a diastolic measurement between 80 mmHg and 90 mmHg, a third bin valued at 8 points can be associated with a systolic measurement between 120 mmHg and 129 mmHg and a diastolic measurement below 80 mmHg, and a fourth bin valued at 10 points can be associated with a systolic measurement below 120 mmHg and a diastolic measurement below 80 mmHg. Other points values or calculations can be used. The blood pressure compliance score, if used, can provide points based on a number of times the user's blood pressure was measured. For example, if no blood pressure measurements were taken, the blood pressure compliance score may be a first number (e.g., 0), but after a first measurement the blood pressure compliance score may be a second number (e.g., 2) and after a second measurement the blood pressure compliance score may be a third number (e.g., 5). Other durations and point values may be used, as well as other measures of compliance. In some cases, the blood pressure score is a combination of the blood pressure measurement score and the blood pressure compliance score. In such an example, a user who measures their blood pressure twice in a day and measures at an average of 123 mmHg over 78 mmHg may receive a blood pressure measurement score of 8 and a blood pressure compliance score of 5, for a total blood pressure score of 13.

In an example, a component score can be an activity score. The activity score can be based on physiological data from block 1102. The activity score can be based at least in part on an activity measurement score, an activity compliance score, or a combination thereof. In an example, physiological data associated with activity (e.g., a count of steps taken during a day) can be received, such as from a wearable device (e.g., a smartwatch or pedometer) or from user input (e.g., fields on a user input interface). Point values can be attributed to different bins based on targets for activity. In an example, a target can be to walk 10,000 steps in a day, and the activity measurement score can be based on what percentage of the target has been accomplished (e.g., 3,000 steps would equate to meeting 30% of the target). In an example, a first bin valued at 0 points can be associated with achieving 0% of the target, a second bin valued at 2 points can be associated with achieving 15% of the target, a third bin valued at 3 points can be associated with achieving 25% of the target, a fourth bin valued at 5 points can be associated with achieving 50% of the target, a fifth bin valued at 8 points can be associated with achieving 75% of the target, and a sixth bin valued at 10 points can be associated with achieving at least 100% of the target. Any number of bins can be used, with any number of associated point values and target thresholds. Other points values or calculations can be used. The activity compliance score, if used, can provide points based on a duration of time the user's activity was measured (e.g., the duration of time the user wore an activity tracking device). For example, if the duration of time the user's activity was measured is less than 10 hours, the activity compliance score may be a first number (e.g., 0), but if the user's activity is measured for at least 10 hours, the activity compliance score may be a second number (e.g., 5). Other durations and point values may be used, as well as other measures of compliance. In some cases, the activity score is a combination of the activity measurement score and the activity compliance score. In such an example, a user who measures their activity for 11 hours and achieves 9,000 out of 10,000 steps may receive an activity measurement score of 8 and an activity compliance score of 5, for a total activity score of 13. In some cases, an activity score can be based on other activity-related measurements, such as intensity of an activity, frequency of an activity, or the like. In some cases, an activity compliance score can be based on other factors, such as the number of consecutive days in which activity was tracked.

In some cases, process 1100 can optionally include receiving medical device usage data at block 1110. Receiving medical device usage data at block 1110 can include receiving data associated with using a medical device, such as respiratory therapy system 120 of FIG. 1. In some cases, medical device usage data received at block 1110 can be received as part of receiving first physiological data received at block 1104, although that need not always be the case.

In some cases, received medical device usage data can be used at block 1112 to generate one or more component scores. For example, the received medical device usage data from block 1110 can include breathing rate data, which can be part of the physiological data leveraged at block 1112 to generate a component score. In some cases, received medical device usage data from block 1110 can be used to modify how one or more component scores are calculated. For example, the way the sleep score is calculated may be adjusted based on whether or not a respiratory system was used during the sleep session. In another example, the use of a respiratory system is simply a part of the calculation of the sleep score.

At block 1118, a total health score associated with the sleep session (e.g., the sleep session associated with the physiological data received at block 1102 and the subjective feedback data received at block 1108) can be calculated using the component scores from block 1112. Calculating the total health score can include adding up the component scores from block 1112 and/or applying the component scores to a formula. In some cases, calculating the total health score can include applying weighting values to each of the component scores, as disclosed in further detail herein. The total health score can be an indication of the overall health of the individual as it relates to a particular sleep session, as determined based on the evaluated components.

At block 1120, the scores can be presented. Presenting a score at block 1120 can include presenting the total health score, presenting one or more component scores, or any combination thereof. In some cases, presenting a total health score can include presenting a numerical score, such as a numerical score between a minimum score and a maximum score (e.g., between 0 and 100, although other minimum scores and maximum scores can be used). The total health score can also be presented in other fashions, such as a display element with an area partially filled in proportional to the total health score (e.g., a display element in the form of a bar filled in 82% representing a health score of 82 out of 100).

In some cases, presenting a component score can include presenting an indication of the component score, an indication of an underlying measurement score, an indication of the underlying measured values, an indication of an underlying compliance score, an indication of underlying metadata used to determine the compliance score, an indication of a target associated with the component, or any combination thereof. In an example, such as the blood pressure measurement indication 560 of FIG. 5B, presenting a blood pressure component score can include presenting the component score as a graphical display element (e.g., bar that is filled in proportionate to the component score), presenting the underlying blood pressure measurement (e.g., "115/80" representing 115 mmHg systolic and 80 mmHg diastolic), and presenting a target (e.g., "<120/80" representing a target maximum of 120 mmHg systolic and/or 80 mmHg diastolic). In this example, the target is also visually displayed as a line in the graphical display element used to present the component score.

In some cases, presenting a score can include presenting a score on a dashboard view, such as dashboard view 500 of FIGS. 5A and 5B. A total health score can be presented at a total health score indication (e.g. total health score indication 530 of FIGS. 5A and 5B) and component scores can be presented at individual component score indications (e.g., sleep score indication 540, sleepiness score indication 550, blood pressure measurement indication 560, and activity measurement indication 570 of FIGS. 5A and 5B).

In some cases, presenting scores at block 1120 can include presenting a total health score in a form that indicates the relative contribution of each component score used to generate the total health score. In some cases, presenting the total health score includes presenting the total health score as an area on a display element (e.g., graph), the area on the display element made up of a set of sub-areas, each of the sub-areas being associated with a respective component score of the set of component scores and sized proportionally to the amount the component score contributed to the total health score. For example, the total health score indication 530 of FIG. 5D depicts the total health score as a ring made up of several segments (e.g., graphical health score component indications 534A-534D of FIG. 5D), each segment associated with a respective component score. In this example, the relative sizes of the ring segments indicate the relative contribution each component score had on the total health score (e.g., as depicted in FIG. 5D, the activity level measurement associated with the fourth graphical indication 534D contributed less to the overall total health score than the sleep score component associated with the first graphical indication 534A, as indicated by the relative sizes of each graphical indication). In some cases, presenting scores at block 1120 can include presenting a numerical indication of the amount each component score contributed to the total health score, such as via component indications 612A-612D of FIG. 6.

In some cases, presenting a score at block 1120 can include presenting a score with an indication of target achievement. The indication of target achievement can be indicative of whether or not a particular component score or total health score has achieved a certain target (e.g., achieved a desired target, such as a healthy threshold), or how far off the score is from the target. In some cases, an indication of target achievement can be in the form of color coding. For example, a component score within a desired range can be presented in a green color, a component score slightly below a desired range can be presented in an orange color, and/or a component score well below a desired range (e.g., below a second threshold), can be presented in a red color. Other types of indications can be used to indicate target achievement.

In some cases, if medical device usage data is received at block 1110, presenting a score at block 1120 can include presenting an indication of whether or not a medical device was used during the sleep session. For example, a therapy indication (e.g., therapy indication 516G of FIG. 5C) can be presented when the medical device usage data received at block 1110 is indicative that therapy was used, or used for a minimum duration, during the sleep session. In such cases, if no medical device usage data is received at block 1110 or the medical device usage data that is received indicates no therapy was used or therapy was used for less than a minimum duration, the therapy indication can indicate no therapy was performed or can be simply not present (e.g., the lack of a therapy indication under date indication 512F of FIG. 5Cs).

In some cases, appropriate blocks of process 1200 can occur repeatedly throughout a day or throughout a duration of time associated with a sleep session. In some cases, block 1112 can be repeated as necessary for one or more components to update one or more component scores as physiological data and/or subjective feedback data continues to be received. Updates to component scores at block 1112 can also cause the total health store to be updated at block 1118 as new data is being considered. For example, during the course of a duration of time adjacent a sleep session, a user may engage in activity that is tracked (e.g., as a cumulative step count) by a wearable device. When process 1200 first occurs in the day, no activity data may be available or the activity component score may be very low, which can be reflected in the total health score and can be indicated when score(s) are presented at block 1120. However, throughout the day, activity-related physiological data can be received at block 1102. In this example, the amount of activity-related physiological data received at block 1102 by late afternoon may be used to generate a particular activity component score, which can be reflected in the total health score and can be indicated when score(s) are presented at block 1120. Thus, as the user continues to engage in actions that would increase the user's activity component score, process 1100 can dynamically update component scores and the total health score. In such cases, a user may be able to perform various actions (e.g., walking more, taking more blood pressure measurements, or other actions) to try and maximize that day's health score, and the user may receive feedback of such actions in the form of seeing the total health score increase after such actions.

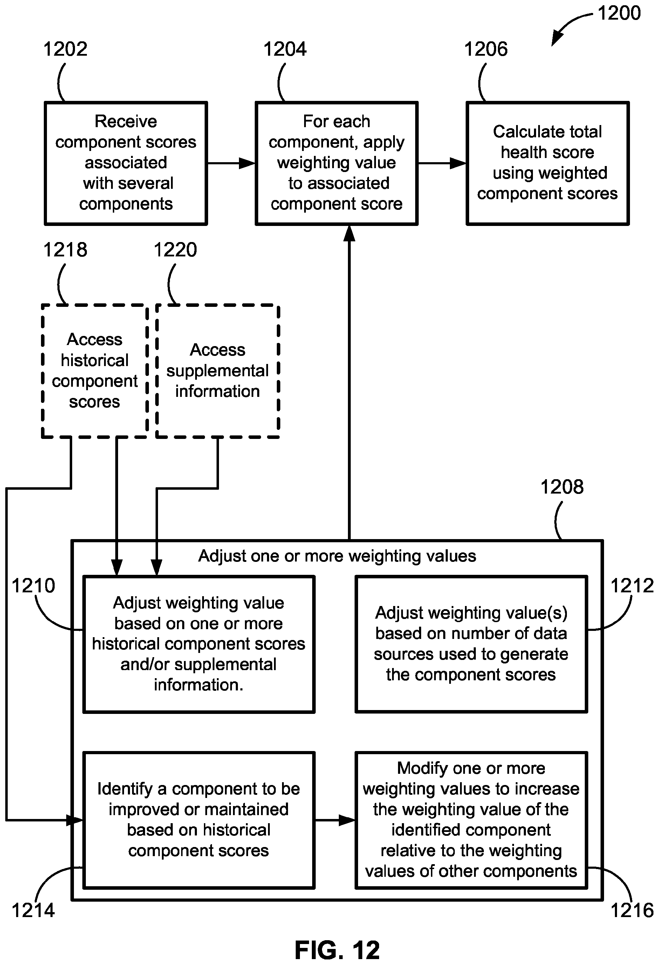

FIG. 12 is a flowchart depicting a process 1200 for calculating a health score from component scores, according to certain aspects of the present disclosure. Process 1200 can be performed by any suitable computing device(s), such as any device(s) of system 100 of FIG. 1. In some cases, process 1200 can be performed by a smartphone, tablet, home computer, or other such device. In some cases, some or all of process 1200 occurs for each iteration of block 1118 of FIG. 11.

At block 1202, component scores associated with several components can be received. Component scores received at block 1202 can be similar to component scores generated at block 1112 of FIG. 11. At block 1204, a weighting value associated with a particular component score (e.g., a weighting value associated with a particular component) can be applied to each respective component score received at block 1202. Applying weighting values to a component score, such as by multiplying the component score by the weighting value, can result in a weighted component score. While described with reference to component scores for illustrative purposes, in some cases, weighting values can be applied to portions of component scores, such as measurement scores and compliance scores (e.g., a measurement score may have a different weighting value than that of a compliance score associated with the same component). At block 1206, the total health score can be calculated using the weighted component scores.

Based on the weighting values, certain components can be emphasized or de-emphasized in the total health score. For example, to emphasize a first component, weighting values associated with any component can be adjusted such that the weighting values of the first component are increased relative to the weighting values of other components. Thus, emphasizing a first component can include increasing the weighting value(s) associated with the first component and/or decreasing the weighting values associated with other components.

At block 1208, one or more weighting values can be adjusted. Weighting values can be adjusted at any suitable time, such as periodically (e.g., hourly, daily, or monthly), on-demand (e.g., upon request by the user), or automatically (e.g., dynamically based on detected trends and/or received physiological data or subjective feedback data). In some cases, adjusting a weighting value at block 1208 can include directly setting or adjusting a weighting value, such as initially setting a weighting value upon programming the system or manually setting a weighting value based on a desire to emphasize certain components in the total health score.

In some cases, adjusting a weighting value at block 1208 can include adjusting a weighting value based on one or more historical component scores and/or supplemental information at block 1210. At optional block 1218, historical component scores can be accessed. Historical component scores can include any component scores associated with previous sleep sessions (e.g., any sleep sessions prior to the sleep session associated with the component scores from block 1202). In some cases, adjusting a weighting value at block 1210 can include analyzing historical component scores to identify one or more trends and/or make one or more inferences, then adjusting one or more weighting values based on the identified trend(s) or inference(s). For example, a user who has recently started using the system and who exhibits low component scores for a particular component may be identified and this information used to adjust (e.g., increase or decrease) the weighting values associated with that particular component.

In some cases, at optional block 1220, supplemental information can be accessed. Supplemental information can include any suitable information usable to adjust a weighting value, such as demographic information associated with the user, medical history information associated with the user, or family health information associated with the user. At block 1210, the supplemental information can be analyzed to determine one or more weighting adjustments. For example, upon first using the system, supplemental information (e.g., user-provided or accessed through other sources) can be analyzed to determine appropriate weighting values for one or more components, permitting the default weighting values to be adjusted and tailored to the user based on the supplemental information. In an example, supplemental information indicating a family history and medical history of high blood pressure may be used to adjust (e.g., increase or decrease) a weighting value associated with a blood pressure component. For example, decreasing the weighting value associated with a blood pressure component may be suitable for someone with a history of and/or genetic disposition for high blood pressure, thus providing a meaningful total health score tailored to that user (e.g., de-emphasizing components that a user may not be able to control). In an alternate example, it may be suitable to increase the weighting value associated with a blood pressure component for someone with a history of and/or genetic disposition for high blood pressure, thus providing additional motivation to improve one's blood pressure.

In some cases, adjusting a weighting value at block 1208 can include adjusting, at block 1212, one or more weighting values based on a number of data sources used to generate the component scores from block 1202. When physiological data and/or subjective feedback data is received and used to generate component scores, such as with respect to process 1100 of FIG. 11, any number of data sources may be used. For example, physiological data may be received from one or more sensors of a respiratory system, a smartphone, and/or a wearable device. The number of data sources used to generate the component scores can be taken into account by adjusting the weighting values for the component scores at block 1212. For example, if a particular component score is normally generated based on three data sources, however two of those data sources were missing for a particular sleep session, the weighting values associated with that component score can be adjusted accordingly (e.g., decreased to de-emphasize potentially less-accurate data). In another example, if a sleep component score relies heavily on data sources from a medical device (e.g., a respiratory system), but that medical device was not used for a particular sleep session, the system can optionally adjust one or more weighting values to de-emphasize the sleep component score for the total health score associated with that sleep session.

In some cases, adjusting a weighting value at block 1208 can include identifying a component to be improved or maintained based on historical component scores at block 1214 and modifying one or more weighting values to increase the weighting value of the identified component relative to the weighting values of the other components at block 1216. At block 1214, historical component scores can be received from block 1218, then analyzed to identify a component to be improved (e.g., urged towards a target) or maintained (e.g., maintained at or around the target). In some cases, identifying a component to be improved or maintained can include identifying a trend associated with that component, although that need not always be the case. In an example, if a user regularly exhibits low activity, the system can identify that the activity component is a component to be improved.

In some cases, identifying a component to be improved or maintained includes ranking some or all components of the set of components associated with the component scores from block 1202. Such ranking can be based on any suitable criteria, such as based on a component's distance from a target, a component's empirical importance to health, a component's subj ective importance to the user, or other criteria.

At block 1216, the identified component from block 1214 can be emphasized in the total health score by increasing weighting values of the identified component relative to the other components. Thus, the component identified to be improved or maintained has a larger impact on the overall total health score, thus motivating the user to improve or maintain that component. Since, in some cases, presenting a score (e.g., presenting a score at block 1120 of FIG. 11) includes presenting an indication of each component's relative contribution to the total health score, a user can easily see what components are important to improve. Since adjustments to weighting values can occur periodically or automatically in some cases, it can be especially helpful for the user to be able to easily see and identify relative contribution of each component to the total health score.

In some cases, adjusting one or more weighting values at block 1208 can include applying a set of component scores to a machine learning algorithm. The machine learning algorithm can be trained to achieve a certain desired result (e.g., a health goal) and can adjust the one or more weighting values to accomplish that desire result. For example, to achieve better blood pressure results in the long run, the machine learning algorithm may be trained to emphasize an activity component until it reaches a certain level or relative level, then switch to emphasizing another component.

As described with reference to FIG. 12, various component scores can be emphasized and de-emphasized through the use of weighting values, which can be adjusted, such as according to various techniques disclosed herein. In some cases, alternative to or in addition to adjusting a weighting value, the way a component score is generated can be adjusted. For example, targets of a component score can be adjusted and/or a formula or algorithm used to generate the component score can be adjusted in a manner similar to how the weighting value can be adjusted (e.g., similarly as described with reference to blocks 1210, 1212, 1214, and 1216. For example, for an individual with a medical history of and/or genetic predisposition for high blood pressure, instead of or in addition to emphasizing or de-emphasizing the blood pressure component score by adjusting weighting values associated with the blood pressure component score, one or more targets (e.g., target ranges) associated with the blood pressure component score or a method of calculating the blood pressure component score (e.g., increasing or decreasing score values associated with each target range) can be adjusted.

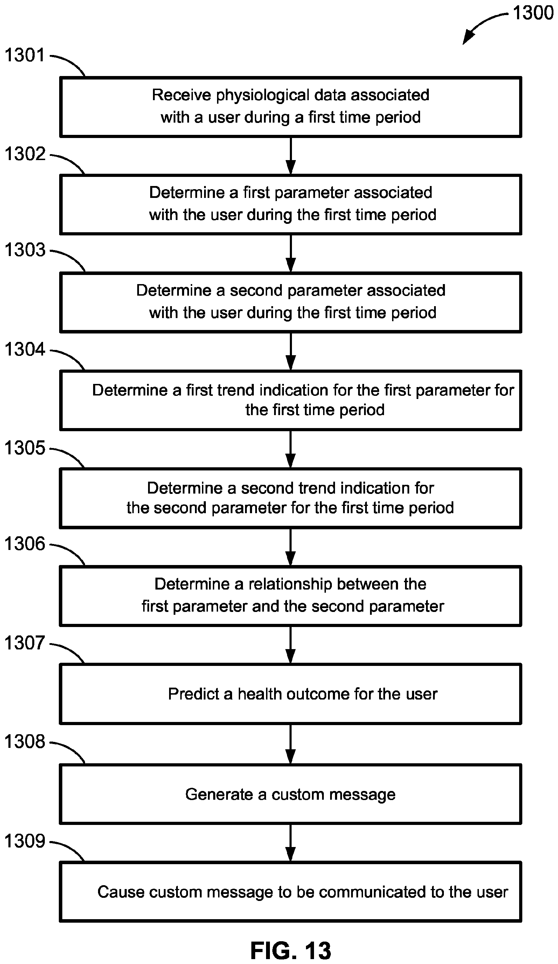

Referring to FIG. 13, a method 1300 for generating a custom message to aid in encouraging a behavioral response, according to some implementations of the present disclosure, is illustrated. One or more steps or aspects of the method 1300 can be implemented using any element or aspect of the system 100 (FIGS. 1-2) described herein.

Step 1301 of the method 1300 includes receiving physiological data associated with a user during a first time period. The physiological data can be generated by one or more of the sensors 130 (FIG. 1) described herein. The received physiological data can indicative of one or more physiological parameters such as, for example, movement, heart rate, heart rate variability, cardiac waveform, respiration rate, respiration rate variability, respiration depth, a tidal volume, an inspiration amplitude, an expiration amplitude, an inspiration-expiration ratio, perspiration, temperature (e.g., ambient temperature, body temperature, core body temperature, surface temperature, etc.), blood oxygenation, photoplethysmography, pulse transmit time, blood pressure, or any combination thereof. The physiological data can be received from the one or more sensors 130 by, for example, the user device 170 described herein, and stored in the memory 114 (FIG. 1).

The first time period includes at least one sleep session. For example, a first portion of the physiological data for the first time period can be associated with a first portion of a first day (e.g., a Monday) before the user begins a sleep session and a second portion of the first day after the user begins a sleep session. If the sleep session extends into the next day (e.g., a Tuesday) before ending, the physiological data for the first time period will also be associated with a portion of the second day. More generally, the first time period can a plurality of days, such as, for example, three days, five days, seven days, fourteen days, twenty-one days, thirty days, etc. In some implementations, the first time period includes at least a portion of each of at least three days. In such implementations, the at least three days can be three consecutive days (e.g., a Monday, Tuesday, and Wednesday) or three non-consecutive days.

Step 1302 of the method 1300 includes determining a first parameter associated with the user based at least in part on a first portion of the received physiological data (step 1301). For example, the control system 110 (FIG. 1) can analyze a portion of the received physiological data (step 1310) to determine the first parameter. Information associated with or describing the determined first parameter can be stored in the memory 114 (FIG. 1).

In some implementations, the first parameter is a sleep-related parameter such as, for example, a sleep score (also referred to as a myAir score), an apnea-hypoapnea index (AHI), an identification of one or more events experienced by the user, a number of events per hour, a pattern of events, a total sleep time, a total time in bed, a wake-up time, a rising time, a hypnogram, a total light sleep time, a total deep sleep time, a total REM sleep time, a number of awakenings, a sleep-onset latency, or any combination thereof. In such implementations, the first parameter is a sleep score such as the ones described in International Publication No. WO 2015/006364.

In some implementations, the determined first parameter (step 1302) or the determined second parameter (1303) is the total health score described herein.

The sleepiness score generally describes the alertness or fatigue of the user following a sleep session. In some implementations, the sleepiness score described above can be determined based at least in part on the received physiological data (step 1301). In such implementations, the determined sleepiness score is an objective measurement of sleepiness. For example, determining the sleepiness score can include determining a reaction time of the user (e.g., in response to a stimulus) and comparing the determined reaction time to a predetermined threshold value to determine whether the user experienced symptoms of impaired cognition during the day. As another example, determining the sleepiness score can include determining an activity level of user during the day using the physiological data and comparing the determined activity level to a predetermined threshold value to determine whether the user experienced symptoms of fatigue during the day. In other implementations, the method 1300 includes receiving subjective feedback from the user for determining the sleepiness score. In such implementations, the sleepiness score is a subjective measurement of sleepiness. For example, the user can indicate their subjective feelings of sleepiness by selecting a number on a scale (e.g., an integer between 0 and 5, where 0 is the sleepiest and 5 is not sleepy at all).

Step 1303 of the method 1300 includes determining a second parameter associated with the user based at least in part a second portion of the received physiological data (step 1301). For example, the control system 110 (FIG. 1) can analyze a portion of the received physiological data (step 1310) to determine the second parameter. Information associated with or describing the determined second parameter can be stored in the memory 114 (FIG. 1). The determined second parameter (step 1303) is different than the determined first parameter (step 1302).

In some implementations, the determined second parameter is a non-sleep-related parameter, such as, for example, a blood pressure measurement (e.g., a systolic measurement, a diastolic measurement, or both), an activity measurement, or a heart-related measurement. The blood pressure measurement can include a systolic component, a diastolic component, or both. The activity level measurement can include a number of steps by the user during the first time period. The heart-related measurement can include an average heart rate during the first time period, a heart rate variability during the first time period, a maximum heart rate during the first time period, or any combination thereof. In some implementations, the determined first parameter or the determined second parameter is a total health score.

In some implementations, one or both of step 1302 and step 1303 include determining a plurality of parameters. For example, step 1302 can include determining a plurality of sleep-related parameters, including a sleep score and a sleepiness score, while step 1303 can include determining a plurality of non-sleep-related parameters, including a blood pressure measurement and an activity level measurement.

In some implementations, the first portion of the physiological data for determining the first parameter (step 1302) is generated by a first one of the one or more sensors 130 (FIG. 1), while the second portion of the physiological data for determining the second parameter (step 1303) is generated by a second one of the one or more sensors 130 that is different than the first sensor. In a first example, the first portion of the physiological data can be generated by the pressure sensor 132 and/or the flow rate sensor 134, which are physically coupled to or integrated in the respiratory therapy system 120. In this first example, the second portion of the physiological data can be generated a sensor that is physically coupled to or integrated in the blood pressure device 180 (e.g., the pressure sensor 132, the PPG sensor 154, etc.) and/or another sensor that is physically coupled to or integrated in the activity tracker 190 (e.g., the motion sensor 138, the PPG sensor 154, etc.). In other implementations, the first portion of the physiological data for determining the first parameter (step 1302) and the second portion of the physiological data for determining the second parameter (step 1303) are generated by the same sensor(s) of the one or more sensors 130.

Step 1304 of the method 1300 includes determining a first trend associated with the determined first parameter (step 1302) based on the first time period. For example, the control system 110 can analyze the determined first parameter and the first time period to determine the first trend. The first trend can be a positive trend, a negative trend, a stable-good trend, or a stable-bad trend, as described herein. The determined first parameter can have a plurality of data points, where each data point corresponds to one day in the time period. For example, the determined first parameter can have a first data point for a first day, a second data point for a second day, and a third data point for a third day. In this example, step 1304 can include determining a fitted line between the first data point, second data point, and third data point and determining a slope of the fitted line to determine the first trend.

Step 1305 of the method 1300 includes determining a second trend associated with the determined second parameter (step 1303) based on the first time period. For example, the control system 110 can analyze the determined second parameter and the first time period to determine the second trend. The second trend can be a positive trend, a negative trend, a stable-good trend, or a stable-bad trend, as described herein. The second trend can be determined in the same or similar manner as the first trend (step 1304).

In some implementations, step 1302, step 1303, step 1304, and step 1305 are performed sequentially. In other implementations, these steps can be performed in alternative sequences. For example, step 1303 can be performed before step 1302, or substantially simultaneously with step 1302. Similarly, as another example, step 1302 and step 1304 can be performed before step 1303 and step 1305, or vice versa.

Step 1306 of the method 1300 includes determining a relationship between the determined first parameter (step 1302) and the determined second parameter (step 1303) based at least in part on the determined first trend (step 1304) and the determined second trend (step 1305). The determined relationship can be determined based on a statistical analysis, such as a correlation between the first parameter and the second parameter or a regression analysis for the first parameter and the second parameter. For example, if the determined first parameter is a sleep score and the determined second parameter is a blood pressure measurement, the determined correlation can indicate that a lower blood pressure measurement (e.g., a measurement that is within a target range) is associated with an improved sleep score (e.g., a sleep score that is within a target range).

Step 1307 of the method 1300 includes predicting a health outcome for the user. The predicted health outcome can be, for example, a life expectancy of the user (e.g., in years), a physical appearance of the user (e.g., to demonstrate predicted aging), progression of a disease, a risk score of stroke, a risk score of blood clotting, a risk score of heart attack, a risk score of weight gain, a risk score of weight loss, or any combination thereof. Communicating the predicted health outcome to the user can further aid in encouraging the behavioral response. For example, if the predicted health outcome is a predicted life expectancy predicted physical appearance of the user, this information can strongly motivate the user to modify their behavior (e.g., CPAP usage, diet, exercise, sleep habits, etc.) to improve the predicted health outcome.

For example, the control system 110 (FIG. 1) can analyze the determined first parameter (step 1302), the determined second parameter (step 1303), the determined first trend (step 1304), the determined second trend (step 1305), the determined relationship (step 1306), or any combination thereof to predict the health outcome for the user. The predicted health outcome can also be determined based at least in part on information in the user profile described herein (e.g., demographic and/or medical information). In some implementations, step 1307 includes using a machine learning algorithm that is trained to receive as an input any combination of the determined first parameter (step 1302), the determined second parameter (step 1303), the determined first trend (step 1304), the determined second trend (step 1305), or the determined relationship (step 1306) and determine as an output the predicted health outcome. In such implementations, the machine learning algorithm can be trained (e.g., via a supervised or unsupervised learning technique) using previously-recorded data associated with user and/or data associated with other individuals.

Step 1307 can further include determining a risk score for at least one of a plurality of health outcomes, such as a risk score of stroke, a risk score of blood clotting, a risk score of heart attack, a risk score of weight gain, a risk score of weight loss, or any combination thereof. Step 1307 can further include determining a trend associated with the risk score for each of the plurality of health outcome to further aid in encouraging the behavioral response by the user.

In some implementations, step 1307 further includes receiving an image of at least a portion (e.g., a face) of the user (e.g., from a photo library on the user device 170). In such implementations, step 1307 also includes generating a modified image of the user based at least in part on the predicted health outcome. For example, step 1307 can include modifying the appearance of the user in the received image to show a change in physical appearance (e.g., aging, wrinkles, weight gain, weight loss, etc.).

Step 1308 of the method 1300 includes generating a custom message to aid in encouraging a behavioral response by the user. The behavioral response can be, for example, using the respiratory therapy system 120 as prescribed, modifying one or more daily habits (e.g., activity level, diet, etc.), one modifying one or more sleep habits (e.g., bedtime, wakeup time, bedtime activities, etc.), or any combination thereof. In some implementations, the control system 110 generates the custom message based at least in part on the determined first parameter (step 1302), the determined second parameter (step 1303), the determined first trend (step 1304), the determined second trend (step 1305), the determined relationship (step 1306), the predicted health outcome (step 1307), or any combination thereof. The generated custom message can include a first indication associated with the determined first parameter, a second indication associated with the determined second parameter, a third indication associated with the determined first trend, a fourth indication associated with the determined second trend, a fifth indication associated with the determined relationship between the first parameter and the second parameter, a seventh indication associated with the predicted health outcome, or any combination thereof. Each of these indications can include, for example, alphanumeric text, image(s), video(s), graphic(s), symbol(s), color(s), or any combination thereof.

The custom message can include information associated with the determined first trend (step 1304) and/or the determined second trend (step 1305) and the first time period (e.g., "sleep score improved in the last three days," "sleep score is worse in the last three days," "sleep score stayed the same in the last three days," "sleep score maintained well in the last three days," etc.). The custom message can also include information associated with the determined relationship (step 1306) and the first time period (e.g., "sleep score is worse while sleepiness is worse during in the last seven days," "sleep score improved while blood pressure improved in the last seven days," "sleep score is worse while blood pressure is worse in the last seven days," etc.).

In some implementations, the generated custom message includes a recommendation, educational information, feedback, or any combination thereof. The recommendation can communicate recommended steps or actions for improving the determined first parameter (step 1302), improving the determined second parameter (step 1303), the predicted health outcome (step 1307), or any combination thereof. The educational information can information describing the determined first parameter (step 1302), improving the determined second parameter (step 1303), the determined relationship between the first parameter and the second parameter (step 1306), the predicted health outcome (step 1307), or any combination thereof (e.g., "Studies have shown a significant reduction in systolic and diastolic blood pressures among sleep apnea patients who were compliant with CPAP therapy for three months (AASM)" or "did you know that episodes of apnea or hypopnea experienced by patients with SDB during sleep can cause a rise in blood pressure?"). The feedback can include positive or negative reinforcement to further aid in encouraging the behavioral response by the user (e.g., "looks like you are on your way, keep up the great work!" or "Well done, you're taking steps towards better health!").

In some implementations, the method 1300 further includes receiving information describing a user-provided objective from the user. The user-provided objective can be, for example, an activity objective (e.g., number of steps), a blood pressure objective (e.g., a target pressure), a sleep objective (e.g., number of hours of sleep, target sleep score, target sleepiness score), or another health-related objective (e.g., weight loss).

In one non-limiting example, the generated custom message includes the following text: "In the last week you didn't sleep as well and your blood pressure increased, did you know that episodes of apnea or hypopnea experienced by patients with SDB during sleep cause a rise in blood pressure? Have you read our tips on how to get a good night's sleep on CPAP?" In another non-limiting example, the generated custom message includes the following text: "In the last week your sleep quality improved and your blood pressure reduced to within the normal range. Studies have shown a significant reduction in systolic and diastolic blood pressures among sleep apnea patients who were compliant with CPAP therapy for three months (AASM), looks like you are on your way, keep up the great work!" In yet another non-limiting example, the generated custom message includes the following text: "On the nights you used CPAP for more than 6 hours, your blood pressure was lower than when you started. Well done, you're taking steps towards better health!"

Step 1309 of the method 1300 includes causing the generated custom message (step 1308) to be communicated to the user subsequent to the first time period. The control system 110 (FIG. 1) can cause the generated custom message to communicated to the user via the display device 172 (e.g., using one or more visual indications) and/or the speaker 142 (e.g., using one or more audio indications). For example, as described above, the control system 110 can cause the custom message 520 to be displayed in the dashboard view 500 on the display device 172 as shown in FIG. 5A.

In some implementations, step 1309 includes causing the generated custom message to be communicated to the user at a predetermined time subsequent to the first time period. The predetermined time can be, for example, a time of day (e.g., 9:00 AM, 1:00 PM, etc.) or time period (e.g., morning, afternoon, evening, etc.). Alternatively, the predetermined time can be a predetermined time subsequent to the first time period (e.g., 2 hours, 8 hours, 1 day, etc.). The predetermined time can be selected to further aid in encouraging the behavioral response by the user.

In some implementations, one or more steps of the method 1300 can be repeated for one or more additional time periods subsequent to the first time period. For example, the method 1300 can further include receiving second physiological data associated with the user during a second time period that is subsequent to the first time period in the same or similar manner as step 1301, determining a third parameter associated with the user based at least in part on a first portion of the second physiological data in the same or similar manner as step 1302, determining a fourth parameter associated with the user based at least in part on a second portion of the second physiological data in the same or similar manner as step 1303, generating a second custom message based at least in part on the determined third parameter, the determined fourth parameter, or both, in the same or similar manner as step 1308, causing the second custom message to be communicated to the user subsequent to the second time period, in the same or similar manner as step 1309. In such implementations, the second custom message can be the same as, or different than, the custom message.

While the method 1300 has been described herein as including each of steps 1301-1309, more or fewer steps can be including in a method for generating one or more custom messages for a user to aid in encouraging a behavioral response. For example, a first alternative method includes step 1301, step 1302, step 1303, step 1308, and step 1309. As another example, a second alternative method includes step 1301, step 1302, step 1303, step 1304, step 1305, step 1308, and step 1309. As yet another example, a second alternative method includes step 1301, step 1302, step 1303, step 1304, step 1305, step 1306, step 1308, and step 1309.

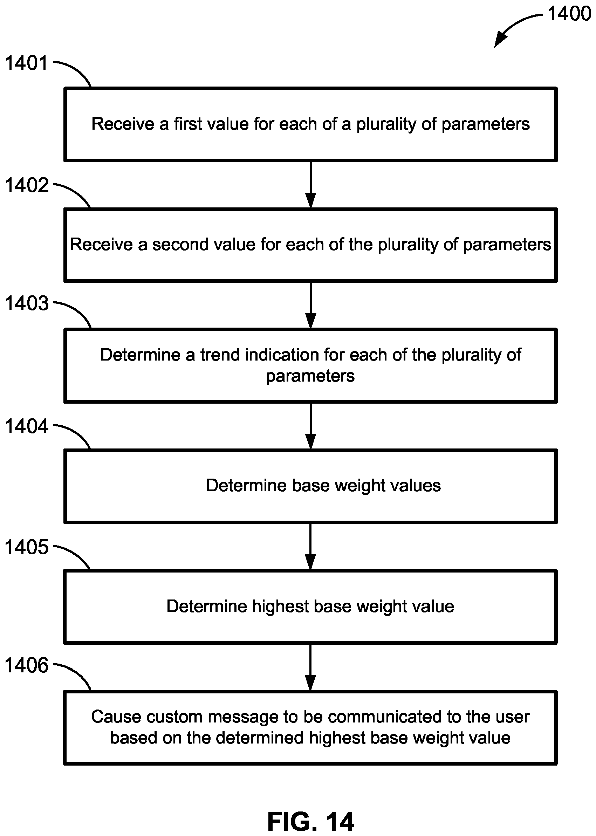

Referring to FIG. 14, a method 1400 for selecting one of a plurality of custom messages to aid in encouraging a behavioral response, according to some implementations of the present disclosure, is illustrated. One or more steps or aspects of the method 1400 can be implemented using any element or aspect of the system 100 (FIGS. 1-2) described herein.

Step 1401 of the method 1400 includes receiving a first value for each of a plurality of parameters. The first values can be received by, for example, the memory 114 (FIG. 1). The plurality of parameters can include a sleep score, a sleepiness score, a blood pressure measurement, an activity level measurement, a total health score, or any combination thereof.

Each of the first values are associated with a user and a first day. In some implementations, the first value for each of the plurality of parameters is a numerical value. For example, first value for the sleep score can be between 0 and 100, the first value for the sleepiness score can be between 0 and 5, the first value for the total health score can be between 0 and 100. The first value for the blood pressure measurement can include a systolic component, a diastolic component, or both. The first value for the activity level measurement can be, for example, a number of steps by the user during the first day (e.g., 1,000 steps, 5,000 steps, 10,000 steps, etc.).

The first values can be determined based at least in part on physiological data generated by at least one of the one or more sensors 130 (FIG. 1) in the same or similar manner as described in step 1302 (FIG. 13) above. In some implementations, the physiological data used to determine the first values for each of the plurality of parameters is generated during at least a portion of the first day and at least a portion of a second day that is immediately subsequent to the first day (e.g., to include a sleep session that begins on the first day and ends on the next day).

Step 1402 of the method 1400 includes receive a second value for each of the plurality of parameters. The second values can be received by, for example, the memory 114 (FIG. 1). The plurality of parameters is the same plurality of parameters for which the first values are received in step 1401. The second values can be determined in the same or similar manners as the first values that are received in step 1401. The second values differ from the first values (step 1401) in that the second values are associated with a second day that is subsequent to the first day. The first day and the second day can be two immediately consecutive days (e.g., a Monday and a Tuesday) or two non-consecutive days (e.g., a Monday and a Friday) with multiple intermediate days therebetween (e.g., 1 intermediate day, 5 intermediate days, 7 intermediate days, 30 intermediate days, 100 intermediate days, etc.).

Step 1403 of the method 1400 includes determining a trend indication for each of the plurality of parameters based at least in part on the first values (step 1401), the second values (step 1402), and a first time period. For example, the control system 110 (FIG. 1) can analyze the first values (step 1401), the second values (step 1402), and the first time period to determine the trend indications. The determined trend indications can be stored in the memory 114 (FIG. 1).

In some implementations, each of the trend indications is determined based on a slope of a fitted line between at least the first value and the second value for each of the plurality of parameters, for example, as shown in FIG. 9 and FIGS. 10A-10B. As described herein, the trend indication can be positive, negative, stable-good, or stable-bad based on the slope of the fitted line. For example, the trend indication can be positive if the slope of the fitted line is greater than about 0.05 and the trend indication can be negative if the slope of the fitted line is less than about -0.05. The trend indication can be stable if the slope of the fitted line is greater than about -0.05 and less than about 0.05. To determine whether the stable trend indication is stable-good or stable-bad, step 1403 also includes comparing the first value and the second value to predetermined healthy threshold values for each of the plurality of parameters. The trend indication is stable-good if the first value and the second value are within a range of predetermined healthy threshold values, or stable-bad if the first value and the second value are not within the range of predetermined healthy threshold values. Exemplary values for the range of predetermined healthy threshold values, according to some implementations of the present disclosure, are provided in Table 1 below.

**Table 1**

| **Parameter** | **Minimum** | **Maximum** |
|---|---|---|
| Sleep Score (myAir) | 70 | 100 |
| Blood Pressure | 115 | 130 |
| Sleepiness | 0 | 2 |
| Activity Level | 1,500 | 30,000 |

Using Table 1 as an example, if the first value for the sleep score parameter is 80 and the second value for the sleep score parameter is 80 (such that the slope of the fitted line between at least the first value and the second value is 0), the determined trend indication is stable-good.

In some implementations, for the range of predetermined healthy threshold values are customized for the user. For example, the range of predetermined healthy threshold values can be customized based on the user profile described herein (e.g., demographic information such as age, medical information, etc.). As described in further detail herein, the range of predetermined healthy threshold values can also be modified in subsequent iterations of the method 1400 based on data associated with the user.

Step 1404 of the method 1400 includes determining a base weight value for each of the plurality of parameters, multiple pairs of the plurality of parameters, the first day, the second day, or any combination thereof. For example, the control system 110 (FIG. 1) can determine the base weight value for each of the plurality of parameters, multiple pairs of the plurality of parameters, the first day, the second day, or any combination thereof. The determined base weight values can be stored in the memory 114 (FIG. 1).

Each of the plurality of parameters, the multiple pairs of the plurality of parameters, the first day, and the second day, are associated with a custom message that can be generated using the method 1300 (FIG. 13) described herein. As described below, the base weight value can be a number (e.g., an integer between 1 and 99) that is used to determine which of these custom messages to be communicated to the user to aid in encouraging a behavioral response. Generally, the base weight values are assigned such that the custom message with the greatest base weight value is the custom message that is most likely to encourage the behavioral response.

In some implementations, each of the determined base weight values are based on a predetermined initial set of base weight values. The predetermined initial set of base weight values can be selected based at least in part on information in the user profile associated with the user, such as, for example, demographic information (e.g., age, gender, etc.) and/or medical information. As described herein, the predetermined initial set of base weight values can be modified based at least in part on user feedback in subsequent iterations of the method 1400.

The base weight values for the first day and the second day can be determined relative to the day that user begins using the system 100 (FIG. 1) (e.g., the day the user first begins using the respiratory therapy system 120). For example, the first day that the user uses the system 100 is associated with a base weight value of 100, the second day that the user uses the system 100 is associated with a base weight value of 99, the third day that the user uses the system 100 is associated with a base weight value of 98, the fifth day that the user uses the system 100 is associated with a base weight value of 70, the tenth day that the user uses the system 100 is associated with a base weight value of 51, the fifteenth day that the user uses the system 100 is associated with a base weight value of 75, the twentieth day that the user uses the system 100 is associated with a base weight value of 51, the twenty-fifth day that the user uses the system 100 is associated with a base weight value of 51, the twenty-ninth day that the user uses the system 100 is associated with a base weight value of 75, the thirtieth day that the user uses the system 100 is associated with a base weight value of 85, the thirty-first day that the user uses the system 100 is associated with a base weight value of 90, the thirty-fifth day that the user uses the system 100 is associated with a base weight value of 51, the thirty-sixth or greater day that the user uses the system 100 is associated with a base weight value of 51. The base weight value for the days between each of these days are interpolated. For example, based on the base weight value of 98 for the third day and the base weight value of 70 for the fifth day, the base weight value for the fourth day is 84.

The base weight values for each of the plurality of parameters can be determined based at least in part on the determined trend indications (step 1403) for each of the plurality of parameters and the first time period that includes at least the first day and the second day. That is, the base weight values for each of the plurality of parameters is a function of both the associated trend indication (e.g., positive, negative, stable-good, or stable-bad) and the length of the first time period (e.g., 3 days, 7 days, 14 days, 21 days, 28 days, etc.). Exemplary base weight values for each of the plurality of parameters for a time period of 3 days, according to some implementations of the present disclosure, are provided in Table 2 below.

**Table 2**

| | **Positive** | **Stable-good** | **Stable-bad** | **Negative** |
|---|---|---|---|---|
| **Sleep Score Base Weight** | 75 | 71 | 64 | 60 |
| **Blood Pressure Base Weight** | 70 | 70 | 70 | 70 |
| **Sleepiness Score Base Weight** | 50 | 50 | 50 | 50 |
| **Activity Level Base Weight** | 10 | 10 | 10 | 10 |

Exemplary base weight values for each of the plurality of parameters for a time period of 7 days, according to some implementations of the present disclosure, are provided in Table 3 below.

**Table 3**

| | **Positive** | **Stable-good** | **Stable-bad** | **Negative** |
|---|---|---|---|---|
| **Sleep Score Base Weight** | 60 | 58 | 53 | 50 |
| **Blood Pressure Base Weight** | 70 | 65 | 55 | 50 |
| **Sleepiness Score Base Weight** | 50 | 50 | 50 | 50 |
| **Activity Level Base Weight** | 10 | 10 | 10 | 10 |

Exemplary base weight values for each of the plurality of parameters for a time period of 14 days, according to some implementations of the present disclosure, are provided in Table 4 below.

**Table 4**

| | **Positive** | **Stable-good** | **Stable-bad** | **Negative** |
|---|---|---|---|---|
| **Sleep Score Base Weight** | 50 | 50 | 50 | 50 |
| **Blood Pressure Base Weight** | 75 | 69 | 56 | 50 |
| **Sleepiness Score Base Weight** | 10 | 10 | 10 | 10 |
| **Activity Level Base Weight** | 10 | 10 | 10 | 10 |

Exemplary base weight values for each of the plurality of parameters for a time period of 21 days, according to some implementations of the present disclosure, are provided in Table 5 below.

**Table 5**

| | **Positive** | **Stable-good** | **Stable-bad** | **Negative** |
|---|---|---|---|---|
| **Sleep Score Base Weight** | 50 | 50 | 50 | 50 |
| **Blood Pressure Base Weight** | 80 | 73 | 58 | 50 |
| **Sleepiness Score Base Weight** | 10 | 10 | 10 | 10 |
| **Activitv Level Base Weight** | 10 | 10 | 10 | 10 |

Exemplary base weight values for each of the plurality of parameters for a time period of 28 days, according to some implementations of the present disclosure, are provided in Table 6 below.

**Table 6**

| | **Positive** | **Stable-good** | **Stable-bad** | **Negative** |
|---|---|---|---|---|
| **Sleep Score Base Weight** | 50 | 50 | 50 | 50 |
| **Blood Pressure Base Weight** | 85 | 76 | 59 | 50 |
| **Sleepiness Score Base Weight** | 10 | 10 | 10 | 10 |
| **Activity Level Base Weight** | 10 | 10 | 10 | 10 |

The base weight values for each of the multiple pairs of the plurality of parameters can be determined based at least in part on the first time period and the determined trend indication associated with each of the plurality of parameters of the one pair of the plurality of parameters. To illustrate, if the plurality of parameters includes the sleep score, the sleepiness score, the blood pressure measurement, and the activity level measurement, the multiple pairs of the plurality of parameters includes: a sleep score and sleepiness score pair, a sleep score and blood pressure measurement pair, a sleep score and activity level pair, a sleepiness score and blood pressure measurement pair, a sleepiness score and activity level pair, a blood pressure measurement and activity level measurement pair, or any combination thereof. That is, the base weight values for each of the multiple pairs plurality of parameters is a function of both the associated trend indication for each parameter in the pair (e.g., positive, negative, stable-good, or stable-bad) and the length of the first time period (e.g., 3 days, 7 days, 14 days, 21 days, 28 days, etc.). Exemplary base weight values for each of the plurality of parameters for a time period of 7 days, according to some implementations of the present disclosure, are provided in Table 7 below.

**Table 7**

| **First Parameter Trend** | **Second Parameter Trend** | **Base Weight Value** |
|---|---|---|
| Sleep Score - Positive | Blood Pressure - Positive | 80 |
| Sleep Score - Positive | Blood Pressure - Negative | 80 |
| Sleep Score - Positive | Sleepiness - Positive | 65 |
| Sleep Score - Positive | Sleepiness - Negative | 70 |
| Sleep Score - Positive | Activity - Positive | 20 |
| Sleep Score - Positive | Activity - Negative | 20 |
| Sleep Score - Negative | Blood Pressure - Positive | 80 |
| Sleep Score - Negative | Blood Pressure - Negative | 80 |
| Sleep Score - Negative | Sleepiness - Positive | 65 |
| Sleep Score - Negative | Sleepiness - Negative | 65 |
| Sleep Score - Negative | Activity - Positive | 20 |
| Sleep Score - Negative | Activity - Negative | 20 |
| Blood Pressure - Positive | Sleepiness - Positive | 50 |
| Blood Pressure - Positive | Sleepiness - Negative | 50 |
| Blood Pressure - Positive | Activity - Positive | 20 |
| Blood Pressure - Positive | Activity - Negative | 20 |
| Blood Pressure - Negative | Sleepiness - Positive | 50 |
| Blood Pressure - Negative | Sleepiness - Negative | 50 |
| Blood Pressure - Negative | Activity - Positive | 20 |
| Blood Pressure - Negative | Activity - Negative | 20 |
| Sleepiness - Positive | Activity - Positive | 20 |
| Sleepiness - Positive | Activity - Negative | 20 |
| Sleepiness - Negative | Activity - Positive | 20 |
| Sleepiness - Negative | Activity - Negative | 20 |

In some implementations, a base weight value for one of the pairs of the plurality of parameters that is greater than 50 is indicative of a positive valence between the parameters in the pair.

Step 1405 of the method 1400 includes determining which of the determined base weight values is greater than all the others of the determined base weight values. For example, the control system 110 (FIG. 1) can analyze each of the determined base weight values to identify the one of the first day, the second day, the one of each of the plurality of parameters, or the one of the multiple pairs of the plurality of parameters that has a base weight value that is greater than all of the other base weight values.

In some examples, step 1405 may result in a tie between two or more base weight values that are greater than all the others of the determined base weight values. In response to determining that the determined base weight value for a first one of the plurality of parameters is equal to the determined base weight value for a second one of the plurality of parameters, step 1405 further includes modifying (i) the determined base weight value associated with the first one of the plurality of parameters based on a first veto multiplier associated with the first one of the plurality of parameters and (ii) the determined based weight value associated with the second one of the plurality of parameters based on a second veto multiplier associated with the second one of the plurality of multipliers. Exemplary veto multiplier values, according to some implementations of the present disclosure, are provided in Table 8 below.

**Table 8**

| **Parameter** | **Veto Multiplier** |
|---|---|
| Sleep Score | 70 |
| Blood Pressure | 60 |
| Sleepiness Score | 45 |
| Activity Level | 33 |

Using the veto multipliers in Table 8 as an example, if the determined base weight value for the sleep score parameter and the determined base weight value for the blood pressure measurement are both 80, step 1405 includes selecting the sleep score parameter because the associated veto multiplier (70) is greater than the associated veto multiplier for the blood pressure parameter (60).

Step 1406 of the method 1400 includes causing a message to be communicated to the user based at least in part on the determined greatest base weight value (step 1405). The custom message is the same as, or similar to, the custom messages generated using the method 1300 (FIG. 13) described above. The control system 110 (FIG. 1) can cause the generated custom message to communicated to the user via the display device 172 (e.g., using one or more visual indications) and/or the speaker 142 (e.g., using one or more audio indications). For example, as described above, the control system 110 can cause the custom message 520 to be displayed in the dashboard view 500 on the display device 172 as shown in FIG. 5A. In some implementations, the custom message is only associated with the one of the first day, second day, one of the plurality of parameters, one of the multiple pairs of the plurality of parameters that is associated with the greatest determined base weights, and not the others of the first day, second day, the plurality of parameters, the multiple pairs of the plurality of parameters that are not associated with the greatest determined base weight.

One or more steps of the method 1400 can be repeated one or more times for one or more time periods subsequent to the first time period. For example, in some implementations, the method 1400 includes receiving a third value for each of the plurality of parameters in the same or similar manner as the first values (step 1401) and/or the second values (step 1402). The third values differ from the first values and the second values in that the third values are associated with a third day that is subsequent to both the first day and the second day. The first day, second day, and third day can be consecutive days (e.g., a Monday, Tuesday, and Wednesday), or non-consecutive days. After receiving the third values, the method 1400 can include determining, for each of the plurality of parameters, a trend indication based at least in part on the first values, the second values, and/or the third values, and a second time period that includes the first day, the second day, and the third day.

As described above, step 1404 of the method 1400 includes determining the base weight values based at least in part on a predetermined initial set of base weight values. In such implementations, where the method 1400 is repeated for the second time period, the method 1400 can include modifying the predetermined initial set of base weight values. Generally, the initial base weight values can be modified such that custom messages that are more likely to encourage the behavioral response are prioritized and caused to be communicated to the user. In such implementations, the base weight values can be modified using a machine learning algorithm.

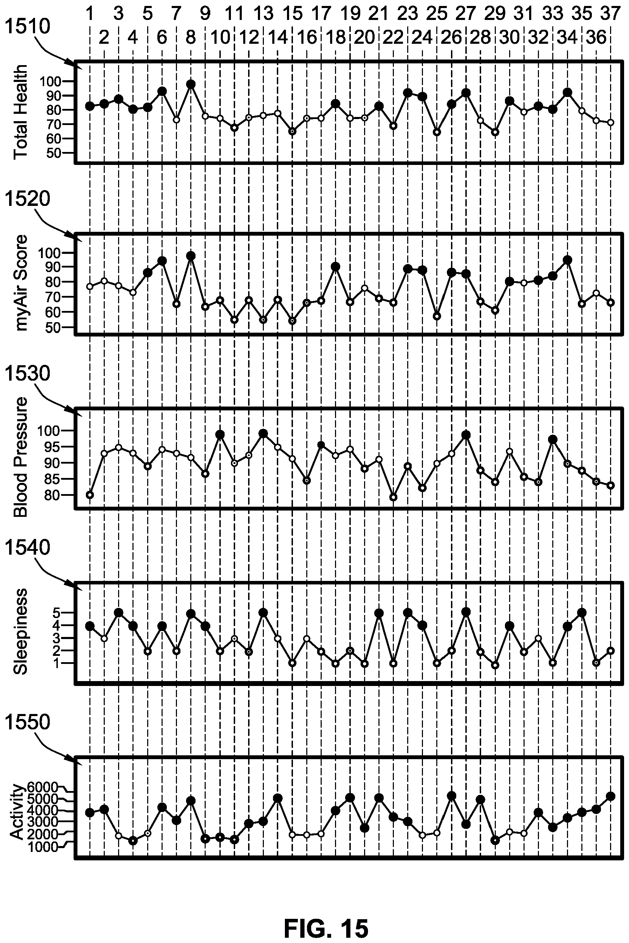

Referring to FIG. 15, exemplary values for a plurality of parameters over a time period of 37 days are illustrated, including values 1510 for a health score parameter, values 1520 for a sleep score parameter, values 1530 for a blood pressure measurement parameter, values 1540 for a sleepiness score parameter, and values 1550 for an activity level parameter (in this example, a number of steps).

Implementing the method 1400 causes a custom message to be communicated to the user for each of the days based on the values 1510-1550 corresponding that day. Table 9 below includes the generated custom message for each day based on the values 1510-1550, the base weight values in Tables 2-7 and the veto multipliers in Table 8.

| **Da y** | **THS** | **Custom Message** |
|---|---|---|
| 1 | 83 | Day 1 message! |
| 2 | 83 | Day 2 message! |
| 3 | 87 | Day 3 message! |
| 4 | 80 | Day 4 message! |
| 5 | 82 | Sleep score (myAir) improved in the last 3 days! |
| 6 | 93 | Sleep score (myAir) improved in the last 3 days! |
| 7 | 73 | Blood pressure improved in the last 3 days! |
| 8 | 97 | Sleep score (myAir) maintained well in the last 3 days! |
| 9 | 75 | Blood pressure improved in the last 3 days! |
| 10 | 74 | Blood pressure maintained well in the last 3 days! |
| 11 | 68 | Blood pressure maintained well in the last 3 days! |
| 12 | 74 | Sleep score (myAir) is worse while sleepiness is worse in the last 7 days. |
| 13 | 76 | Blood pressure improved in the last 3 days! |
| 14 | 77 | Sleep score (myAir) is worse while sleepiness is worse in the last 7 days. |
| 15 | 66 | Sleep score (myAir) is worse while blood pressure is worse in the last 7 days. |
| 16 | 74 | Blood pressure improved in the last 3 days! |
| 17 | 74 | Sleep score (myAir) improved while blood pressure improved in the last 7 days. |
| 18 | 83 | Sleep score (myAir) improved in the last 3 days! |
| 19 | 74 | Blood pressure maintained well in the last 3 days! |
| 20 | 75 | Blood pressure is worse in the last 3 days! |
| 21 | 83 | Blood pressure improved in the last 3 days! |
| 22 | 70 | Blood pressure maintained well in the last 21 days! |
| 23 | 91 | Sleep score (myAir) improved in the last 3 days! |
| 24 | 88 | Sleep score (myAir) improved in the last 3 days! |
| 25 | 65 | Blood pressure maintained well in the last 21 days! |
| 26 | 84 | Sleep score (myAir) maintained well in the last 3 days! |
| 27 | 91 | Sleep score (myAir) maintained well in the last 3 days! |
| 28 | 73 | Blood pressure improved in the last 3 days! |
| 29 | 65 | Sleep score (myAir) is worse while blood pressure is worse in the last 7 days. |
| 30 | 86 | Day 30 message! |
| 31 | 79 | Day 31 message! |
| 32 | 82 | Blood pressure improved in the last 14 days! |
| 33 | 80 | Sleep score (myAir) improved while blood pressure improved in the last 7 days. |
| 34 | 91 | Blood pressure maintained well in the last 28 days! |
| 35 | 79 | Blood pressure maintained well in the last 28 days! |
| 36 | 73 | Sleep score (myAir) is worse while blood pressure is worse in the last 7 days. |
| 37 | 72 | Blood pressure improved in the last 28 days! |

While the method 1400 has been described herein as including each of steps 1401-1406, more or fewer steps can be including in a method for selecting one of a plurality of custom messages to be communicated to a user to aid in encouraging a behavioral response. For example, a first alternative method includes step 1401, step 1402, step 1403, step 1404, and step 1405. As another example, a second alternative method includes step 1403, step 1404, step 1405, and step 1406.

While the present disclosure has been described with reference to one or more particular embodiments or implementations, those skilled in the art will recognize that many changes may be made thereto without departing from the scope of the present disclosure Each of these implementations and obvious variations thereof is contemplated as falling within the scope of the present disclosure. It is also contemplated that additional implementations according to aspects of the present disclosure may combine any number of features from any of the implementations described herein. The invention is defined by the appended claims.

## Claims

1. A computer-implemented method (1100) comprising:
receiving (1102) physiological data associated with a sleep session, the physiological data including first physiological data (1104) collected during the sleep session and second physiological data (1106) collected during a duration adjacent the sleep session;
receiving (1108) subjective feedback data associated with the sleep session, the subjective feedback data including an indication of sleepiness;
generating (1112) a set of component scores based on the physiological data and the subjective feedback data;
calculating (1118) a total health score associated with the sleep session using the set of component scores; and
presenting (1120) the total health score.

2. The computer-implemented method (1100) of claim 1, wherein, for at least one of the set of component scores, the generating the component score includes:
determining (1114) a measurement score associated with one or more measurements associated with a component being scored;
determining (1116) a compliance score based on a number of measurement instances or a duration of measurement associated with the one or more measurements associated with the component being scored; and
calculating the component score based on the measurement score and the compliance score.

3. The computer-implemented method (1100) of claim 1 or claim 2, further comprising receiving (1110) medical device usage data, the medical device usage data being associated with usage of a medical device during the sleep session, the generating the set of component scores being further based on the medical device usage data; wherein, optionally, the presenting the total health score includes presenting an indication of whether or not the medical device was used during the sleep session.

4. The computer-implemented method (1100) of any one of claims 1 to 3, wherein each component score in the set of component scores is associated with a weighting value (1204), and wherein the calculating the total health score includes, for each component score in the set of component scores, applying (1206) the weighting value to the component score;
wherein, optionally, the method further comprises adjusting (1208) one or more of the weighting values based on a number of data sources (1212) used to generate the set of component scores, and/or adjusting one or more of the weighting values based on one or more historical component scores (1210), wherein, further optionally, the adjusting the one or more of the weighting values includes:
identifying (1214) a component to be improved or maintained based on the one or more historical component scores, the component represented by one component score of the set of component scores; and
modifying (1216) the one or more weighting values to increase a weighting value associated with the one of the set of component scores relative to one or more weighting values associated with other components of the set of component scores.

5. The computer-implemented method (1100) of claim 4, further comprising adjusting one or more of the weighting values by applying the set of component scores to a machine learning algorithm; and/or
the method further comprises:
receiving (1220) demographic information, medical history information, or family health information; and
adjusting (1208, 1210) one or more of the weighting values based on the demographic information, the medical history information, the family health information, or any combination thereof.

6. The computer-implemented method (1100) of any one of claims 1 to 5, wherein the set of component scores includes a sleepiness score based on the subjective feedback data;
wherein, optionally, the sleepiness score is based at least in part on a sleepiness measurement score selected from a range based on the subjective feedback data, and/or the sleepiness score is based at least in part on a sleepiness compliance score that is based on whether or not the subjective feedback data is provided.

7. The computer-implemented method (1100) of any one of claims 1 to 6, wherein the set of component scores includes a blood pressure score based on the second physiological data;
wherein, optionally, the blood pressure score is based at least in part on a blood pressure measurement score that is based at least in part on one or more blood pressure measurements taken during the duration adjacent to the sleep session, and/or
the blood pressure score is based at least in part on a blood pressure compliance score that is based at least in part on a number of times one or more blood pressure measurements were taken during the duration adjacent to the sleep session.

8. The computer-implemented method (1100) of any one of claims 1 to 7, wherein the set of component scores includes an activity score based on the second physiological data;
wherein, optionally, the activity score is based at least in part on an activity measurement score that is based at least in part on a value associated with an amount of activity tracked during the duration adjacent to the sleep session, and/or
the activity score is based at least in part on an activity compliance score that is based at least in part on a length of time activity was tracked during the duration adjacent to the sleep session.

9. The computer-implemented method (1100) of any one of claims 1 to 8, wherein the set of component scores includes a sleep score based on the first physiological data;
wherein, optionally, the sleep score is based at least in part on a sleep measurement score that is based at least in part on a value associated with a quality of sleep during the sleep session, and/or the sleep score is based at least in part on a sleep compliance score that is based at least in part on whether or not the first physiological data was collected during the sleep session.

10. The computer-implemented method (1100) of claim 9, further comprising receiving (1110) medical device usage data, the sleep score being calculated using the medical device usage data; and
wherein, optionally, (i) the calculating the sleep score comprises:
determining at least one sub-score selected from the group consisting of:
determining a usage score representing a duration of time the medical device was used during the sleep session based on the medical device usage data,
determining an interface score representing an effectiveness of a user interface seal of the medical device during the sleep session based on the medical device usage data, and
determining an interruption score representing a number and/or duration of times the user interface was removed during the sleep session based on the medical device usage data; and
using the determined sub-score to calculate the sleep score, and/or (ii) the calculating the sleep score further includes determining an event score representing a number of events that occurred during the sleep session based on the medical device usage data to calculate the sleep score, wherein, optionally, the event score is further combined with one or more of the usage score, the interface score, and the interruption score to calculate the sleep score.

11. The computer-implemented method (1100) of any one of claims 1 to 10, further comprising presenting each of the set of component scores; and/or
presenting, for at least one of the set of components scores, a target value associated with the component score; and/or
presenting, for each of the set of component scores, an indicator of an amount the component score contributed to the total health score, wherein, optionally, the presenting the total health score includes presenting the total health score as an area on a graph, the area on the graph made up of a set of sub-areas, wherein each of the sub-areas is associated with a respective component score of the set of component scores and is sized proportionally to the amount the component score contributed to the total health score.

12. The computer-implemented method (1100) of any one of claims 1 to 11, further comprising presenting, for each of the set of component scores, an indicator representing a deviation from a target value associated with the component score.

13. The computer-implemented method (1100) of any one of claims 1 to 12,
(a) wherein the duration adjacent the sleep session is bounded by a previous sleep session and the sleep session, or
(b) wherein the duration adjacent the sleep session is bounded by the sleep session and a subsequent sleep session, or
(c) wherein the duration adjacent the sleep session includes (i) a pre-sleep duration before the sleep session, (ii) a post-sleep duration after the sleep session, or both (i) and (ii).

14. The computer-implemented (1100) method of claim 1, further comprising:
receiving a set of existing component scores associated with the sleep session or a previous sleep session;
generating a set of updated component scores based on the set of existing component scores and the received data;
calculating the total health score associated with the sleep session using the set of updated component scores;
wherein, optionally, the receiving data associated with the sleep session comprises:
receiving the physiological data associated with the sleep session, the physiological data including the first physiological data collected during the sleep session and the second physiological data collected during the duration adjacent the sleep session; and
receiving the subjective feedback data associated with the sleep session, and/or
wherein the calculating the total health score comprises updating an existing total health score, and wherein the presenting the total health score comprises updating a presentation of an existing total health score using the total health score.

15. A system (100) comprising:
a control system (110) including one or more processors (112); and
a memory (114) having stored thereon machine readable instructions;
wherein the control system (110) is coupled to the memory (114), and the computer-implemented method of any one of claims 1 to 14 is implemented when the machine executable instructions in the memory are executed by at least one of the one or more processors (112) of the control system (110).

16. A computer program product comprising instructions which, when executed by a computer, cause the computer to carry out the method of any one of claims 1 to 14.

## Patentansprüche

1. Computerimplementiertes Verfahren (1100), umfassend:
Empfangen (1102) von physiologischen Daten, die mit einer Schlafsitzung assoziiert sind, wobei die physiologischen Daten erste physiologische Daten (1104), die während der Schlafsitzung erfasst werden, und zweite physiologische Daten (1106) umfassen, die während einer an die Schlafsitzung angrenzenden Dauer erfasst werden;
Empfangen (1108) subjektiver Feedbackdaten, die mit der Schlafsitzung assoziiert sind, wobei die subjektiven Feedbackdaten eine Angabe von Schläfrigkeit umfassen;
Erzeugen (1112) eines Satzes von Komponentenbewertungen basierend auf den physiologischen Daten und den subjektiven Feedbackdaten;
Berechnen (1118) einer mit der Schlafsitzung assoziierten Gesamtgesundheitsbewertung unter Verwendung des Satzes von Komponentenbewertungen; und
Darstellen (1120) der Gesamtgesundheitsbewertung.

2. Computerimplementiertes Verfahren (1100) nach Anspruch 1, wobei das Erzeugen der Komponentenbewertung für mindestens eine des Satzes von Komponentenbewertungen umfasst:
Bestimmen (1114) einer Messungsbewertung, die mit einer oder mehreren Messungen assoziiert ist, die mit einer Komponente assoziiert sind, die bewertet wird;
Bestimmen (1116) einer Konformitätsbewertung basierend auf einer Anzahl von Messinstanzen oder einer Messdauer, die mit der einen oder den mehreren Messungen assoziiert ist, die mit der Komponente assoziiert sind, die bewertet wird; und
Berechnen der Komponentenbewertung basierend auf der Messungsbewertung und der Konformitätsbewertung.

3. Computerimplementiertes Verfahren (1100) nach Anspruch 1 oder 2, ferner umfassend ein Empfangen (1110) von Nutzungsdaten einer medizinischen Vorrichtung, wobei die Nutzungsdaten der medizinischen Vorrichtung mit der Nutzung einer medizinischen Vorrichtung während der Schlafsitzung assoziiert sind, wobei das Erzeugen des Satzes von Komponentenbewertungen ferner auf den Nutzungsdaten der medizinischen Vorrichtung basiert; wobei optional das Darstellen der Gesamtgesundheitsbewertung ein Darstellen einer Angabe umfasst, ob die medizinische Vorrichtung während der Schlafsitzung verwendet wurde oder nicht.

4. Computerimplementiertes Verfahren (1100) nach einem der Ansprüche 1 bis 3, wobei jede Komponentenbewertung in dem Satz von Komponentenbewertungen mit einem Gewichtungswert (1204) assoziiert ist und wobei das Berechnen der Gesamtgesundheitsbewertung für jede Komponentenbewertung in dem Satz von Komponentenbewertungen ein Anwenden (1206) des Gewichtungswerts auf die Komponentenbewertung umfasst;
wobei optional das Verfahren ferner ein Anpassen (1208) eines oder mehrerer der Gewichtungswerte basierend auf der Anzahl von Datenquellen (1212), die zum Erzeugen des Satzes von Komponentenbewertungen verwendet wird, und/oder Anpassen eines oder mehrerer der Gewichtungswerte basierend auf einer oder mehreren historischen Komponentenbewertungen (1210) umfasst, wobei ferner optional das Anpassen des einen oder der mehreren der Gewichtungswerte umfasst:
Identifizieren (1214) einer Komponente, die verbessert oder aufrechterhalten werden soll, basierend auf der einen oder den mehreren historischen Komponentenbewertungen, wobei die Komponente durch eine Komponentenbewertung des Satzes von Komponentenbewertungen dargestellt wird; und
Modifizieren (1216) des einen oder der mehreren Gewichtungswerte, um einen Gewichtungswert, der mit der einen des Satzes von Komponentenbewertungen assoziiert ist, relativ zu einem oder mehreren Gewichtungswerten zu erhöhen, die mit anderen Komponenten des Satzes von Komponentenbewertungen assoziiert sind.

5. Computerimplementiertes Verfahren (1100) nach Anspruch 4, ferner umfassend ein Anpassen eines oder mehrerer der Gewichtungswerte durch Anwenden des Satzes von Komponentenbewertungen auf einen Maschinenlernalgorithmus; und/oder
das Verfahren ferner umfasst:
Empfangen (1220) von demografischen Informationen, Informationen zur Krankengeschichte oder Informationen zur Familiengesundheit; und
Anpassen (1208, 1210) eines oder mehrerer der Gewichtungswerte basierend auf den demografischen Informationen, den Informationen zur Krankengeschichte, den Informationen zur Familiengesundheit oder einer beliebigen Kombination davon.

6. Computerimplementiertes Verfahren (1100) nach einem der Ansprüche 1 bis 5, wobei der Satz von Komponentenbewertungen eine Schläfrigkeitsbewertung umfasst, die auf den subjektiven Feedbackdaten basiert;
wobei optional die Schläfrigkeitsbewertung zumindest teilweise auf einer Schläfrigkeitsmessungsbewertung basiert, die aus einem Bereich ausgewählt wird, der auf den subjektiven Feedbackdaten basiert, und/oder die Schläfrigkeitsbewertung zumindest teilweise auf einer Schläfrigkeitskonformitätsbewertung basiert, die darauf basiert, ob die subjektiven Feedbackdaten bereitgestellt werden oder nicht.

7. Computerimplementiertes Verfahren (1100) nach einem der Ansprüche 1 bis 6, wobei der Satz von Komponentenbewertungen eine Blutdruckbewertung umfasst, die auf den zweiten physiologischen Daten basiert;
wobei optional die Blutdruckbewertung zumindest teilweise auf einer Blutdruckmessungsbewertung basiert, die zumindest teilweise auf einer oder mehreren Blutdruckmessungen basiert, die während der an die Schlafsitzung angrenzenden Dauer vorgenommen werden, und/oder
wobei die Blutdruckbewertung zumindest teilweise auf einer Blutdruckkonformitätsbewertung basiert, die zumindest teilweise auf einer Anzahl von Malen basiert, die eine oder mehrere Blutdruckmessungen während der an die Schlafsitzung angrenzenden Dauer vorgenommen wurden.

8. Computerimplementiertes Verfahren (1100) nach einem der Ansprüche 1 bis 7, wobei der Satz von Komponentenbewertungen eine Aktivitätsbewertung umfasst, die auf den zweiten physiologischen Daten basiert;
wobei optional die Aktivitätsbewertung zumindest teilweise auf einer Aktivitätsmessungsbewertung basiert, die zumindest teilweise auf einem Wert basiert, der mit einem Aktivitätsgrad assoziiert ist, der während der an die Schlafsitzung angrenzenden Dauer beobachtet wurde, und/oder
wobei die Aktivitätsbewertung zumindest teilweise auf einer Aktivitätskonformitätsbewertung basiert, die zumindest teilweise auf einer Zeitdauer basiert, in der Aktivität während der an die Schlafsitzung angrenzenden Dauer beobachtet wurde.

9. Computerimplementiertes Verfahren (1100) nach einem der Ansprüche 1 bis 8, wobei der Satz von Komponentenbewertungen eine Schlafbewertung umfasst, die auf den ersten physiologischen Daten basiert;
wobei optional die Schlafbewertung zumindest teilweise auf einer Schlafmessungsbewertung basiert, die zumindest teilweise auf einem Wert basiert, der mit einer Schlafqualität während der Schlafsitzung assoziiert ist, und/oder die Schlafbewertung zumindest teilweise auf einer Schlafkonformitätsbewertung basiert, die zumindest teilweise darauf basiert, ob die ersten physiologischen Daten während der Schlafsitzung gesammelt wurden oder nicht.

10. Computerimplementiertes Verfahren (1100) nach Anspruch 9, ferner umfassend ein Empfangen (1110) von Nutzungsdaten einer medizinischen Vorrichtung, wobei die Schlafbewertung unter Verwendung der Nutzungsdaten der medizinischen Vorrichtung berechnet wird; und
wobei optional (i) das Berechnen der Schlafbewertung umfasst:
Bestimmen mindestens einer Teilbewertung, die ausgewählt wird aus der Gruppe bestehend aus:
Bestimmen einer Nutzungsbewertung, die eine Zeitdauer darstellt, die die medizinische Vorrichtung während der Schlafsitzung verwendet wurde, basierend auf den Nutzungsdaten der medizinischen Vorrichtung,
Bestimmen einer Schnittstellenbewertung, die eine Wirksamkeit einer Benutzerschnittstellendichtung der medizinischen Vorrichtung während der Schlafsitzung darstellt, basierend auf den Nutzungsdaten der medizinischen Vorrichtung, und
Bestimmen einer Unterbrechungsbewertung, die eine Anzahl und/oder eine Zeitdauer darstellt, die die Benutzerschnittstelle während der Schlafsitzung entfernt wurde, basierend auf den Nutzungsdaten der medizinischen Vorrichtung; und
Verwenden der bestimmten Teilbewertungen zum Berechnen der Schlafbewertung und/oder (ii) wobei das Berechnen der Schlafbewertung ferner ein Bestimmen einer Ereignisbewertung, die eine Anzahl von Ereignissen darstellt, die während der Sitzung eintrat, basierend auf den Nutzungsdaten der medizinischen Vorrichtung zum Berechnen der Schlafbewertung umfasst, wobei optional die Ereignisbewertung ferner mit einer oder mehreren der Nutzungsbewertung, der Schnittstellenbewertung und der Unterbrechungsbewertung kombiniert wird, um die Schlafbewertung zu berechnen.

11. Computerimplementiertes Verfahren (1100) nach einem der Ansprüche 1 bis 10, ferner umfassend ein Darstellen jeder des Satzes von Komponentenbewertungen; und/oder
Darstellen eines Zielwerts, der mit der Komponentenbewertung assoziiert ist, für mindestens eine des Satzes von Komponentenbewertungen; und/oder
Darstellen eines Indikators eines Betrags, den die Komponentenbewertung zur Gesamtgesundheitsbewertung beitrug, für jede des Satzes von Komponentenbewertungen, wobei optional das Darstellen der Gesamtgesundheitsbewertung ein Darstellen der Gesamtgesundheitsbewertung als Bereich auf einem Graphen umfasst, wobei der Bereich auf dem Graphen aus einem Satz von Teilbereichen besteht, wobei jeder der Teilbereiche mit einer jeweiligen Komponentenbewertung des Satzes von Komponentenbewertungen assoziiert und proportional zu dem zur Gesamtgesundheitsbewertung beigetragenen Betrag bemessen ist.

12. Computerimplementiertes Verfahren (1100) nach einem der Ansprüche 1 bis 11, ferner umfassend ein Darstellen eines Indikators, der eine Abweichung von einem mit der Komponentenbewertung assoziierten Zielwert darstellt, für jede des Satzes von Komponentenbewertungen.

13. Computerimplementiertes Verfahren (1100) nach einem der Ansprüche 1 bis 12,
a) wobei die an die Schlafsitzung angrenzende Dauer durch eine vorherige Schlafsitzung und die Schlafsitzung begrenzt wird,
b) wobei die an die Schlafsitzung angrenzende Dauer durch die Schlafsitzung und nachfolgende Schlafsitzung begrenzt wird, oder
c) wobei die an die Schlafsitzung angrenzende Dauer (i) eine Vor-Schlaf-Dauer vor der Schlafsitzung, (ii) eine Nach-Schlaf-Dauer nach der Schlafsitzung oder sowohl (i) als auch (ii) umfasst.

14. Computerimplementiertes Verfahren (1100) nach Anspruch 1, ferner umfassend:
Empfangen eines Satzes von bestehenden Komponentenbewertungen, der mit der Schlafsitzung oder einer vorherigen Schlafsitzung assoziiert ist;
Erzeugen eines Satzes von aktualisierten Komponentenbewertungen basierend auf dem Satz von bestehenden Komponentenbewertungen und den empfangenen Daten;
Berechnen einer mit der Schlafsitzung assoziierten Gesamtgesundheitsbewertung unter Verwendung des Satzes von aktualisierten Komponentenbewertungen;
wobei optional das Empfangen von Daten, die mit der Schlafsitzung assoziiert sind, umfasst:
Empfangen der mit der Schlafsitzung assoziierten physiologischen Daten, wobei die physiologischen Daten die ersten physiologischen Daten, die während der Schlafsitzung erfasst werden, und die zweiten physiologischen Daten umfassen, die während der an die Schlafsitzung angrenzenden Dauer erfasst werden; und
Empfangen der mit der Schlafsitzung assoziierten subjektiven Feedbackdaten und/oder wobei das Berechnen der Gesamtgesundheitsbewertung ein Aktualisieren einer bestehenden Gesamtgesundheitsbewertung umfasst und wobei das Darstellen der Gesamtgesundheitsbewertung ein Aktualisieren einer Darstellung einer bestehenden Gesamtgesundheitsbewertung unter Verwendung der Gesamtgesundheitsbewertung umfasst.

15. System (100), umfassend:
ein Steuersystem (110), das einen oder mehrere Prozessoren (112) umfasst; und
einen Arbeitsspeicher (114), der maschinenlesbare Anweisungen darauf gespeichert aufweist:
wobei das Steuersystem (110) mit dem Arbeitsspeicher (114) gekoppelt ist und das computerimplementierte Verfahren nach einem der Ansprüche 1 bis 14 implementiert wird, wenn die maschinenausführbaren Befehle im Arbeitsspeicher von mindestens einem des einen oder der mehreren Prozessoren (112) des Steuersystems (110) ausgeführt werden.

16. Computerprogrammprodukt, umfassend Anweisungen, die bei Ausführung durch einen Computer den Computer zum Durchführen des Verfahrens nach einem der Ansprüche 1 bis 14 veranlassen.

## Revendications

1. Procédé mis en oeuvre par ordinateur (1100) comprenant :
la réception (1102) de données physiologiques associées à une session de sommeil, les données physiologiques incluant des premières données physiologiques (1104) collectées pendant la session de sommeil et des secondes données physiologiques (1106) collectées pendant une durée adjacente à la session de sommeil ;
la réception (1108) de données de rétroaction subjective associées à la session de sommeil, les données de rétroaction subjective incluant une indication de somnolence ;
la génération (1112) d'un ensemble de scores de composantes sur la base des données physiologiques et des données de rétroaction subjective ;
le calcul (1118) d'un score de santé global associé à la session de sommeil en utilisant l'ensemble de scores de composantes ; et
la présentation (1120) du score de santé global.

2. Procédé mis en oeuvre par ordinateur (1100) selon la revendication 1, dans lequel, pour au moins l'un de l'ensemble de scores de composantes, la génération du score de composante inclut :
la détermination (1114) d'un score de mesure associé à une ou plusieurs mesures associées à une composante en cours de notation ;
la détermination (1116) d'un score de conformité sur la base d'un nombre d'instances de mesure ou d'une durée de mesure associée à la ou aux plusieurs mesures associées à la composante en cours de notation ; et
le calcul du score de composante sur la base du score de mesure et du score de conformité.

3. Procédé mis en oeuvre par ordinateur (1100) selon la revendication 1 ou la revendication 2, comprenant en outre la réception (1110) de données d'utilisation de dispositif médical, les données d'utilisation de dispositif médical étant associées à l'utilisation d'un dispositif médical pendant la session de sommeil, la génération de l'ensemble de scores de composantes étant en outre basée sur les données d'utilisation de dispositif médical ; dans lequel, facultativement, la présentation du score de santé global inclut la présentation d'une indication indiquant si le dispositif médical a été utilisé ou non pendant la session de sommeil.

4. Procédé mis en oeuvre par ordinateur (1100) selon l'une quelconque des revendications 1 à 3, dans lequel chaque score de composante dans l'ensemble de scores de composantes est associé à une valeur de pondération (1204), et dans lequel le calcul du score de santé global inclut, par chaque score de composante dans l'ensemble de scores de composantes, l'application (1206) de la valeur de pondération au score de composante ;
dans lequel, facultativement, le procédé comprend en outre l'ajustement (1208) d'une ou plusieurs des valeurs de pondération sur la base d'un nombre de sources de données (1212) utilisées pour générer l'ensemble de scores de composantes, et/ou l'ajustement d'une ou plusieurs des valeurs de pondération sur la base d'un ou plusieurs scores de composantes historiques (1210), dans lequel, en outre facultativement, l'ajustement de la ou des plusieurs valeurs de pondération comprend :
l'identification (1214) d'une composante à améliorer ou à maintenir sur la base du ou des plusieurs scores de composantes historiques, la composante étant représentée par un score de composante de l'ensemble de scores de composantes ; et
la modification (1216) de la ou des plusieurs valeurs de pondération pour augmenter une valeur de pondération associée à l'un de l'ensemble de scores de composantes par rapport à une ou plusieurs valeurs de pondération associées à d'autres composantes de l'ensemble de scores de composantes.

5. Procédé mis en oeuvre par ordinateur (1100) selon la revendication 4, comprenant en outre l'ajustement d'une ou plusieurs des valeurs de pondération en appliquant l'ensemble de scores de composantes à un algorithme d'apprentissage automatique ; et/ou
le procédé comprend en outre :
la réception (1220) d'informations démographiques, d'informations sur les antécédents médicaux ou des informations sur la santé familiale ; et
l'ajustement (1208, 1210) d'une ou plusieurs des valeurs de pondération sur la base des informations démographiques, des informations sur les antécédents médicaux, des informations sur la santé familiale, ou de toute combinaison de celles-ci.

6. Procédé mis en oeuvre par ordinateur (1100) selon l'une quelconque des revendications 1 à 5, dans lequel l'ensemble de scores de composantes inclut un score de somnolence basé sur les données de rétroaction subjective ;
dans lequel, facultativement, le score de somnolence est basé au moins en partie sur un score de mesure de somnolence sélectionné dans une plage basée sur les données de rétroaction subjective, et/ou le score de somnolence est basé au moins en partie sur un score de conformité de somnolence qui est basé sur la founriture ou non des données de rétroaction subjective.

7. Procédé mis en oeuvre par ordinateur (1100) selon l'une quelconque des revendications 1 à 6, dans lequel l'ensemble de scores de composantes inclut un score de pression artérielle basé sur les secondes données physiologiques ;
dans lequel, facultativement, le score de pression artérielle est basé au moins en partie sur un score de mesure de pression artérielle qui est basé au moins en partie sur une ou plusieurs mesures de pression artérielle prises pendant la durée adjacente à la session de sommeil, et/ou
le score de pression artérielle est basé au moins en partie sur un score de conformité de pression artérielle qui est basé au moins en partie sur le nombre de fois où une ou plusieurs mesures de pression artérielle ont été prises pendant la durée adjacente à la session de sommeil.

8. Procédé mis en oeuvre par ordinateur (1100) selon l'une quelconque des revendications 1 à 7, dans lequel l'ensemble de scores de composantes comprend un score d'activité basé sur les secondes données physiologiques ;
dans lequel, facultativement, le score d'activité est basé au moins en partie sur un score de mesure d'activité qui est basé au moins en partie sur une valeur associée à une quantité d'activité suivie pendant la durée adjacente à la session de sommeil, et/ou
le score d'activité est basé au moins en partie sur un score de conformité d'activité qui est basé au moins en partie sur le temps pendant lequel l'activité a été suivie pendant la durée adjacente à la session de sommeil.

9. Procédé mis en oeuvre par ordinateur (1100) selon l'une quelconque des revendications 1 à 8, dans lequel l'ensemble de scores de composantes comprend un score de sommeil basé sur les premières données physiologiques ;
dans lequel, facultativement, le score de sommeil est basé au moins en partie sur un score de mesure de sommeil qui est basé au moins en partie sur une valeur associée à une qualité de sommeil pendant la session de sommeil, et/ou le score de sommeil est basé au moins en partie sur un score de conformité de sommeil qui est basé au moins en partie sur le fait que les premières données physiologiques ont été collectées ou non pendant la session de sommeil.

10. Procédé mis en oeuvre par ordinateur (1100) selon la revendication 9, comprenant en outre la réception (1110) de données d'utilisation de dispositif médical, le score de sommeil étant calculé en utilisant les données d'utilisation de dispositif médical ; et
dans lequel, facultativement, (i) le calcul du score de sommeil comprend :
la détermination d'au moins un sous-score sélectionné dans le groupe constitué de :
la détermination d'un score d'utilisation représentant une durée pendant laquelle le dispositif médical a été utilisé pendant la session de sommeil sur la base des données d'utilisation de dispositif médical,
la détermination d'un score d'interface représentant une efficacité d'un joint d'interface utilisateur du dispositif médical pendant la session de sommeil sur la base des données d'utilisation de dispositif médical, et
la détermination d'un score d'interruption représentant un nombre et/ou une durée de fois où l'interface utilisateur a été supprimée pendant la session de sommeil sur la base des données d'utilisation de dispositif médical ; et
l'utilisation du sous-score déterminé pour calculer le score de sommeil, et/ou (ii) le calcul du score de sommeil inclut en outre la détermination d'un score d'événement représentant le nombre d'événements survenus pendant la session de sommeil sur la base des données d'utilisation de dispositif médical pour calculer le score de sommeil, où, facultativement, le score d'événement est en outre combiné avec un ou plusieurs parmi le score d'utilisation, le score d'interface et le score d'interruption pour calculer le score de sommeil

11. Procédé mis en oeuvre par ordinateur (1100) selon l'une quelconque des revendications 1 à 10, comprenant en outre la présentation de chacun de l'ensemble de scores de composantes ; et/ou
la présentation, pour au moins l'un des scores de composantes, d'une valeur cible associée au score de composante ; et/ou
la présentation, pour chacun de l'ensemble de scores de composantes, d'un indicateur d'une quantité de contribution du score de composante au score de santé global, où, facultativement, la présentation du score de santé global inclut la présentation du score de santé global sous la forme d'une zone sur un graphique, la zone sur le graphique étant constituée d'un ensemble de sous-zones, où chacune des sous-zones est associée à un score de composante respectif de l'ensemble de scores de composantes et est dimensionnée proportionnellement à la quantité de contribution du score de composante au score de santé global.

12. Procédé mis en oeuvre par ordinateur (1100) selon l'une quelconque des revendications 1 à 11, comprenant en outre la présentation, pour chacun de l'ensemble de scores de composantes, d'un indicateur représentant un écart par rapport à une valeur cible associée au score de composante.

13. Procédé mis en oeuvre par ordinateur (1100) selon l'une quelconque des revendications 1 à 12,
a) dans lequel la durée adjacente à la session de sommeil est limitée par une session de sommeil précédente et la session de sommeil, ou
b) dans lequel la durée adjacente à la session de sommeil est limitée par la session de sommeil et une session de sommeil ultérieure, ou
c) dans lequel la durée adjacente à la session de sommeil inclut (i) une durée de pré-sommeil avant la session de sommeil, (ii) une durée de post-sommeil après la session de sommeil, ou les deux (i) et (ii).

14. Procédé mis en oeuvre par ordinateur (1100) selon la revendication 1, comprenant en outre :
la réception d'un ensemble de scores de composantes existants associés à la session de sommeil ou à une session de sommeil précédente ;
la généraion d'un ensemble de scores de composantes mis à jour sur la base de l'ensemble de scores de composantes existants et des données reçues ;
le calcul du score de santé global associé à la session de sommeil en utilisant l'ensemble de scores de composantes mis à jour ;
dans lequel, facultativement, les données de réception associées à la session de sommeil comprennent :
la réception des données physiologiques associées à la session de sommeil, les données physiologiques incluant les premières données physiologiques collectées pendant la session de sommeil et les secondes données physiologiques collectées pendant la durée adjacente à la session de sommeil ; et
la réception des données de rétroaction subjective associées à la session de sommeil, et/ou dans lequel le calcul du score de santé global comprend la mise à jour d'un score de santé global existant, et dans lequel la présentation du score de santé global comprend la mise à jour d'une présentation d'un score de santé global existant en utilisant le score de santé global.

15. Système (100) comprenant :
un système de commande (110) incluant un ou plusieurs processeurs (112) ; et
une mémoire (114) dans laquelle sont stockées des instructions lisibles par machine ;
dans lequel le système de commande (110) est couplé à la mémoire (114), et le procédé mis en oeuvre par ordinateur selon l'une quelconque des revendications 1 à 14 est mis en oeuvre lorsque les instructions exécutables par machine dans la mémoire sont exécutées par au moins l'un du ou des plusieurs processeurs (112) du système de commande (110).

16. Produit programme informatique comprenant des instructions qui, lorsqu'elles sont exécutées par un ordinateur, amènent l'ordinateur à réaliser le procédé selon l'une quelconque des revendications 1 à 14.
